# EUROPEAN PATENT APPLICATION

(11) **EP 4 585 050 A1**
(43) Date of publication of application: **16.07.2025**
(21) Application number: 25151172.1
(22) Date of filing: 10.01.2025
(51) Int. Cl.: A23L 25/00, A23L 33/105, A23L 33/115, A23L 33/14, A23L 33/21, A23L 33/00, A61P 1/00, A61P 3/04, A61P 3/06, A61P 3/08

(54) **FOOD COMPOSITION AND USES THEREOF**

(30) Priority: 12.01.2024 EP 24386003; 20.06.2024 GB 202407177
(71) Applicant: ZOE Limited, London SE1 7RW (GB)
(72) Inventor: AMATI, Federica, London, SE1 7RW (GB); HADJIGEORGIOU, George, London, SE1 7RW (GB); WOLF, Jonathan, London, SE1 7RW (GB); SPECTOR, Tim, London, SE1 7RW (GB); BERMINGHAM, Kate, London, SE1 7RW (GB); BERRY, Sarah, London, SE1 7RW (GB)
(74) Representative: J A Kemp LLP

(57) **Abstract**

The present invention relates to a prebiotic food composition comprising or consisting of at least 15 of the following ingredients: flaxseed, chia seeds, sunflower seeds, pumpkin seeds, hemp seeds, red lentil flakes, grape seed, almonds, hazelnuts, walnuts, chicory root inulin fibre, puffed quinoa, white mushroom, thyme, onion, parsley, turmeric, cumin, Lion's Mane, Reishi, Chaga, Shiitake, Cordyceps, Maitake, Tremella, rosemary, garlic, red beetroot flakes, carrot flakes, nutritional yeast flakes, baobab powder, and buckthorn, as well as its uses.

## Description

### Field of the Invention

The present invention relates to a food composition and methods of use thereof.

### Background to the Invention

There is a considerable body of evidence highlighting the close association between the microbiome and host health, with the roles performed by the microbiome spanning many functions (Flint et al., Proceedings of the Nutrition Society, 2015;74(1), 13-22; Valdes et al., BMJ. 2018;361:k2179). Firstly, intestinal microbiota are capable of digesting nutrients otherwise inaccessible to humans through host gut physiology, to produce short-chain fatty acids, which are the primary energy source for intestinal epithelial cells (Morrison et al., Gut Microbes, 2016;7(3): 189-200). Secondly, the microbiome is part of the bi-directional relationship with host immunity and is involved in human immune homeostasis and immunological recognition that is both innate and adaptive (Shreiner et al. Curr Opin, Gastroenterol. 2015;31(1):69-75.). Moreover, dysbiosis of the gut microbiome has been reported in many diseased states, including irritable bowel disease, inflammatory bowel disease, gut infections, cardiovascular disease, and psychological conditions such as depression and anxiety.

The differences observed between host gut microbiome communities between healthy and disease states, and between different dietary states, has led to an increase in the use of dietary modulations to influence microbiome composition, both in research and in commercial contexts (Peng et al., Comprehensive Reviews in Food Science e Food Safety, 2020; 19(4): 1908-1933). Two particular groups of gut-active compounds of recent interest are prebiotics (providing a direct source of nutrition that stimulate host-beneficial microbiota as they are indigestible to the host) and probiotics (providing a direct source of live microorganisms that may potentially colonise the gut after reaching the large intestine). Both compounds can be found naturally in foods; for example, probiotic lactic acid bacteria are found in fermented dairy products like yoghurt and cheese, while many foods contain prebiotic compounds in the form of diverse fibres.

However, the single main deficiency in most western diets is dietary fibre. For example, 95% of the American population are fibre deficient. Dietary fibre is essential for the function of a healthy digestive system and a healthy gut microbiome. Dietary fibre improves the production of postbiotic nutrients by gut microbes, slows the absorption of nutrients from food, helping to maintain healthy body weight, and normalises bowel movements by regulating intestinal function and promoting normal stool formation. Dietary fibre also improves gut health by influencing gut microbiome diversity, e.g. by promoting the proliferation of a variety of beneficial gut bacteria. Low-fibre diets have been linked to a number of conditions such as constipation, diarrhoea, heart disease, metabolic diseases and colorectal cancer.

A number of food supplement products have recently been put on the market to provide a single daily dose including a combination of different vitamins and minerals, as well as ingredients designed to optimise the gut microbiome and boost the immune system. However, many of these products lack important nutrients and are not optimally formulated to support user health. For example, AG1 (Athletic Greens (EU) Limited) is a powdered product that is mixed with water to be drunk. Using such supplement products may encourage users to skip meals or to eat less healthily. The processing required for formulating such products may also reduce the nutritional benefits of the ingredients. There is a requirement for improved food composition products.

### Summary of the Invention

The present inventors have developed a minimally processed wholefood composition containing diverse ingredients designed to beneficially impact general health and in particular gut microbiome health.

Accordingly, the invention provides a food composition comprising at least 3 of the following: (a) at least one source of dietary fibre, (b) at least one source of polyphenols, (c) at least one plant protein, (d) at least one source of alpha linolenic acid (ALA), and (e) at least one source of phytosterols, wherein the composition comprises at least one nut or seed. In certain embodiments, the invention provides a food composition comprising:
(a) at least two sources of dietary fibre, wherein the sources of dietary fibre are obtained from two or more of: flaxseed, chia seed, sunflower seeds, red lentil flakes, chicory root inulin fibre, nutritional yeast flakes, almonds, hazelnuts, walnuts, brazil nuts, puffed quinoa, puffed buckwheat and red beetroot flakes;

and at least 2 of the following:
   (b) at least one source of polyphenols, wherein the source of polyphenols is obtained from one or more of mushroom powder, thyme, onion powder, parsley, rosemary, turmeric, cumin, flaxseed, baobab powder, grape seed powder, garlic, buckthorn powder, red beetroot flakes, and carrot flakes;
   (c) at least one plant protein;
   (d) at least one source of alpha linolenic acid (ALA); and
   (e) at least one source of phytosterols, wherein the source of phytosterols is obtained from one or more of sunflower seeds, pumpkin seeds, hemp seed, almonds, hazelnuts, walnuts, and brazil nuts,
wherein the composition comprises at least one nut or seed.

There is a relationship between nut consumption and lower cardiovascular disease (CVD) risk (Arnesen et al. Food Nutr Res. 2023;67:10.29219). Tree nut snack consumption in particular is also associated with better diet quality and lower CVD risk factors in the UK adult population (Dikariyanto et al. Public Health Nutr. 2020;23(17):3160-3169). Dietary fibre slows the absorption of nutrients from food, helping to maintain healthy body weight, normalises bowel movements by regulation of intestinal function, improves gut health by influencing gut microbiome diversity, e.g. by promoting the proliferation of beneficial gut bacteria, and reduces the risk of constipation, diarrhoea, heart disease, metabolic diseases and colorectal cancer. A diet which contains a variety of polyphenol rich foods (such as the Med Diet) is associated with reduced risk of CVD.

In one embodiment, the food composition does not contain a sweetener. Compositions containing sweeteners are more likely to be consumed with other sweet food items, which are typically unhealthy. Artificial sweeteners and non-sugar sweeteners can be up to 700 times sweeter than natural sugar. Inclusion of sweeteners in food compositions can lead to a craving for other sweet or sugary foods, which are typically unhealthy. Research suggests that artificial sweeteners are associated with higher risk of stroke, heart disease and death overall. Accordingly, food compositions without sweeteners are better for overall health.

In one embodiment, the food composition is savoury. This encourages the consumer to ingest the food composition with other savoury foods, which are typically healthier than sweet or sugary foods.

In one embodiment, the food composition is suitable for sprinkling on top of a food item. This means that the food composition is more likely to be consumed with other whole foods. Whole food consumption has been shown to improve glycaemic control, maximise the benefits of a whole food matrix throughout the digestive tract, provide a source of dietary fibre, and benefit the gut microbiome. In addition, the food composition of the invention is eaten together with a meal, and is not used as a substitute for a balanced diet. Many existing food compositions are designed to provide the nutrients contained in whole foods, thereby replacing the source of whole foods. For example, omega-3 fatty acid supplements are often consumed in place of the natural source of omega-3 e.g. fish, to the overall detriment of health. This is a particular problem with powdered supplements that are drunk - they may encourage users to skip meals on the assumption they are receiving nutrients in the supplement drink. Powdered supplements also lack a structural food matrix and so they do not activate the body's satiety signals, leading to increased energy consumption and over-eating.

In one embodiment, the structural matrix of at least one of (a)-(e) of the food composition is maintained. Maintenance of the structural matrix results in reduced postprandial glycemia and overall improvement in glycaemic control. Accordingly, the compositions of the invention are improved over compositions that are milled or powdered to ensure that they can be mixed with water or other liquids, such as milk or juice, before being consumed by drinking. The drawback of such food compositions is that milling or powdering ingredients destroys their food matrix. Ingredients that have a preserved original structure (i.e. ingredients which are not processed, or ingredients which are subjected to minimal processing techniques such as nibbing), expose their nutrients within a food matrix (Murillo et al. Front Nutr. (2022) 9:1025993). These foods require more energy to break down, and this process takes longer, with postprandial glycemia and lipid absorption being reduced. Studies have shown the importance of maintaining food structure in order to improve glycaemic control. For example, the food matrix protects nutrients by making them less accessible to the digestion process and absorption, a fact that leads to a lower rise in blood glucose levels (Miao and Hamaker, Annu Rev Food (2021) 12:169-91).

In one embodiment, at least one of (a)-(e) of the food composition is nibbed so as to maintain its structural matrix. Many of the ingredients of the food composition disclosed herein are too large or fibrous to be consumed in their natural form. However, it is undesirable to mechanically break down the ingredients (e.g. by milling or powdering) as this destroys the structural food matrix. Nibbing of ingredients is therefore useful for reducing the size of the ingredients to bite-size chunks, without destroying the structural food matrix, i.e. nibbed ingredients may be sprinkled on top of food items but they have a maintained structural matrix.

In one embodiment, the food composition contains ingredients that are not powdered. Milling, powdering or finely grinding ingredients destroys their food matrix.

In one embodiment, the food composition comprises ingredient particles or fragments that are at least 1mm in diameter, for example at least 2mm or at least 5mm in diameter. Inclusion of such larger particles or fragments ensures that a food composition cannot be drunk with water and must be sprinkled on a food item, which encourages maintenance of a healthy diet. In certain embodiments, the food composition comprises at least two ingredients, such as at least 3, 4 or 5 ingredients that are present in particles or fragments that are at least 1m, 2mm or 5mm in diameter. Generally, the larger particles or fragments will be nuts and/or seeds.

In one embodiment, the food composition is a wholefood composition. Whole foods have been shown to improve glycaemic control, provide a source of dietary fibre, and benefit the gut microbiome.

In one embodiment, the food composition does not contain an anti-caking agent. Anti-caking agents such as silicon dioxide may lead to gastrointestinal issues and irritation. By providing a whole food composition that is suitable for sprinkling on top of food, the composition of the invention does not require anti-caking agents that may be required for powdered supplements.

In one embodiment, the food composition does not contain an additive.

In one embodiment, the food composition is a prebiotic food supplement.

In one embodiment, the food composition comprises at least 3, 4 or 5 different sources of each of (a)-(e). This is advantageous because the food composition can provide a variety of different ingredients e.g. from a variety of different plants, in a single daily dose. In addition, increased diversity of sources can support increased diversity of bacterial species, supporting microbiome diversity.

In one embodiment, the food composition comprises at least one source of dietary fibre which is obtained from one or more plants. The source of dietary fibre may be obtained from one or more of: flaxseed, chia seed, sunflower seeds, red lentil flakes, chicory root inulin fibre, nutritional yeast flakes, almonds, hazelnuts, walnuts, brazil nuts, puffed quinoa, puffed buckwheat and red beetroot flakes. This is useful because different plants contain different plant fibres, and a diversity of fibre is beneficial for the gut microbiome. Red lentils in particular are a rich source of fibre and there is evidence to suggest that they may be able to improve blood sugar responses after meals. Chicory root inulin fibre in particular is a source of fibre regarded to have prebiotic effects on gut microbiota, promoting the abundances of certain bacteria (Bifidobacterium, Lactobacillus, and Faecalibacterium prausnitzii).

In one embodiment, the food composition comprises at least one source of polyphenols which is one or more spices and herbs. The source of polyphenols may be selected from the group consisting of mushroom powder, thyme, onion powder, parsley, rosemary, turmeric and cumin. The mushroom powder may comprise one or more of Lions Mane, Reishi, Chaga, Shiitake, Cordyceps, Maitake, Tremella. Studies using spice/herb mixes that contain similar ingredients have suggested that increased intakes are associated with modulations in gut microbiota composition, and some spice/herb studies have shown associations with favourable effects on other markers of health, such as blood pressure and inflammation (in adults at risk of cardiometabolic diseases) (Petersen et al. Am J Clin Nutr. 2021; 114(6):1936-1948; Oh et al. Am J Clin Nutr. 2022;115(1):61-72).

In one embodiment, the food composition comprises at least one source of polyphenols which one or more of: flaxseed, baobab powder, grape seed powder, garlic, buckthorn powder, red beetroot flakes, and carrot flakes. Grape seed powder in particular is a source of polyphenols with antioxidant properties. Carrot flakes in particular contain chlorogenic acid. Research suggests that chlorogenic acid can play an important role in regulating glycolipids metabolism (Yu et al., Asia Pac J Clin Nutr. 2022; 31 (4): 602-610*).* Garlic contains a variety of compounds that have been shown to improve cardiovascular disease risk factors, which includes improved cholesterol levels, fasting blood glucose and blood pressure. The spice mix used in the food composition is based on a mix used in a clinical trial which showed significant beneficial improvement in gut microbiome composition (Khine WWT, Haldar S, De Loi S, Lee YK. A single serving of mixed spices alters gut microflora composition: a dose-response randomised trial. Sci Rep. 2021 May 28;11(1):11264. doi: 10.1038/s41598-021-90453-7. PMID: 34050197; PMCID: PMC8163817.).

In one embodiment, the food composition comprises at least one plant protein obtained from one or more of the group consisting of: quinoa, red lentils, nuts, seeds and buckwheat.

In one embodiment, the food composition comprises at least one plant protein obtained from one or more of: puffed quinoa and puffed buckwheat.

In one embodiment, the food composition comprises at least one source of alpha-linolenic acid (ALA), which is one or more seeds. The source of ALA may be selected from the group consisting of: flaxseed and chia seeds.

In one embodiment, the food composition comprises at least one source of phytosterols, which is one or more of seeds and nuts. The source of phytosterols may be selected from the group consisting of: sunflower seeds, pumpkin seeds, hemp seed, almonds, hazelnuts, walnuts, and brazil nuts. These nuts and seeds provide a good source of phytosterols, as well as healthy fats and phenolics.

In one embodiment, the food composition comprises or consists of flaxseed, chia seeds, sunflower seeds, pumpkin seeds, hemp seeds, red lentil flakes, grape seed, almonds, hazelnuts, walnuts, chicory root inulin fibre, puffed quinoa, white mushroom, thyme, onion, parsley, turmeric, cumin, Lion's Mane, Reishi, Chaga, Shiitake, Cordyceps, Maitake, Tremella, rosemary, garlic, red beetroot flakes, carrot flakes, nutritional yeast flakes, baobab powder, and buckthorn.

The invention also provides a method of supplementing the dietary needs of a human, comprising administering to the human the food composition of the invention.

The invention also provides a method of benefitting the gut health and the health of the gut microbiome of a human using the food composition of the invention, wherein the food composition is ingested by the human.

The invention also provides a method of reducing the risk of disease in a human using the food composition of the invention, wherein the food composition is ingested by the human.

The invention also provides a method of improving the general well-being of a human using the food composition of the invention, wherein the food composition is ingested by the human.

The invention also provides a method of controlling or treating obesity in a human using the food composition of the invention, wherein the food composition is ingested by the human.

The invention also provides a method of reducing inflammation in a human using the food composition of the invention, wherein the food composition is ingested by the human. In one aspect, the method of reducing inflammation involves reducing inflammatory markers.

The invention also provides a method of improving the glycaemic profile of a human using the food composition of the invention, wherein the food composition is ingested by the human.

The invention also provides a method of improving the lipaemic profile of a human using the food composition of the invention, wherein the food composition is ingested by the human.

The invention also provides a method of treating gastrointestinal symptoms, for example, constipation and bloating, using the food composition of the invention, wherein the food composition is ingested by the human. The invention also provides a method of treating irritable bowel syndrome using the food composition of the invention, wherein the food composition is ingested by the human.

The invention also provides a method of improving one or more of the following: bowel function of a human, mood of a human, skin quality of a human, satiety of a human or sleep quality of a human, using the food composition of the invention, wherein the food composition is ingested by the human.

The invention also provides a method of strengthening the immune system of a human using the food composition of the invention, wherein the food composition is ingested by the human.

According to the method of the invention, the food composition of the invention is ingested by the human at a daily dosage of about 15g. In one embodiment, the daily dosage comprises about 5g of fibre.

According to the method of the invention, the food composition of the invention is sprinkled on top of a food item.

### Brief Description of the Figures

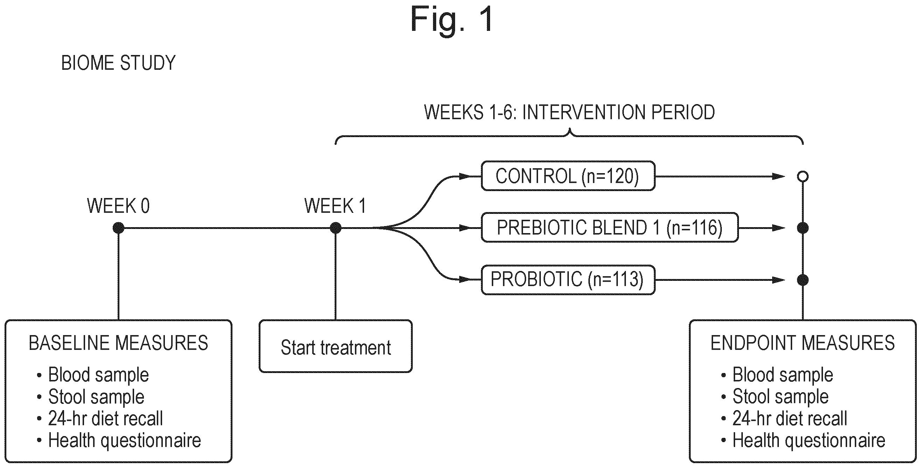

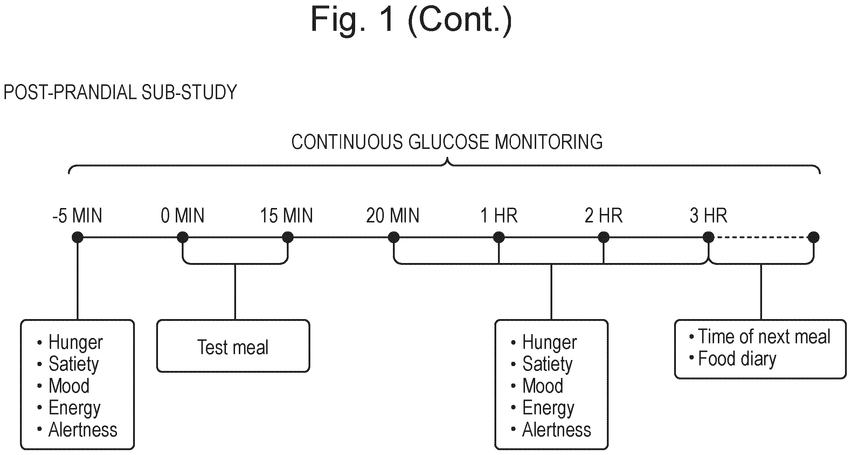

**Figure 1** summarises the BIOME study design (upper panel) and the post-prandial sub-study (lower panel). Participants received a study kit via postal delivery containing materials necessary for completing study measurements prior to the baseline (week 0) and endpoint (week 7). All procedures were conducted by participants in their homes.

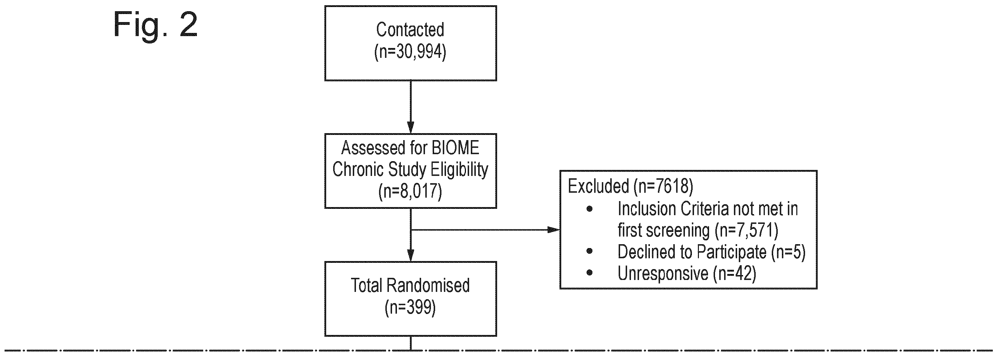

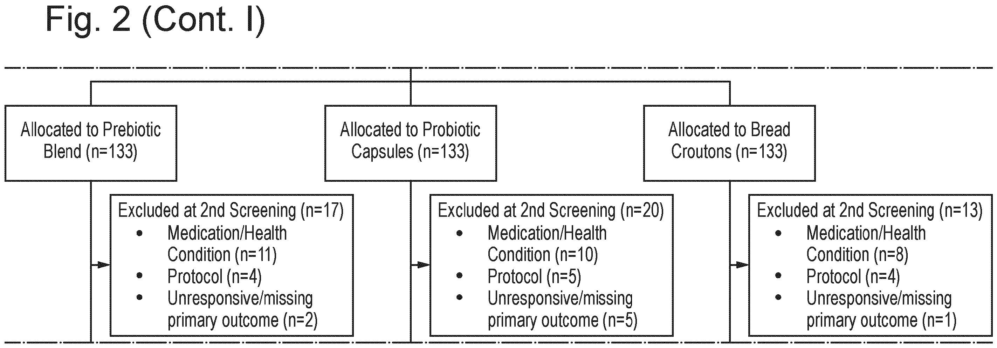

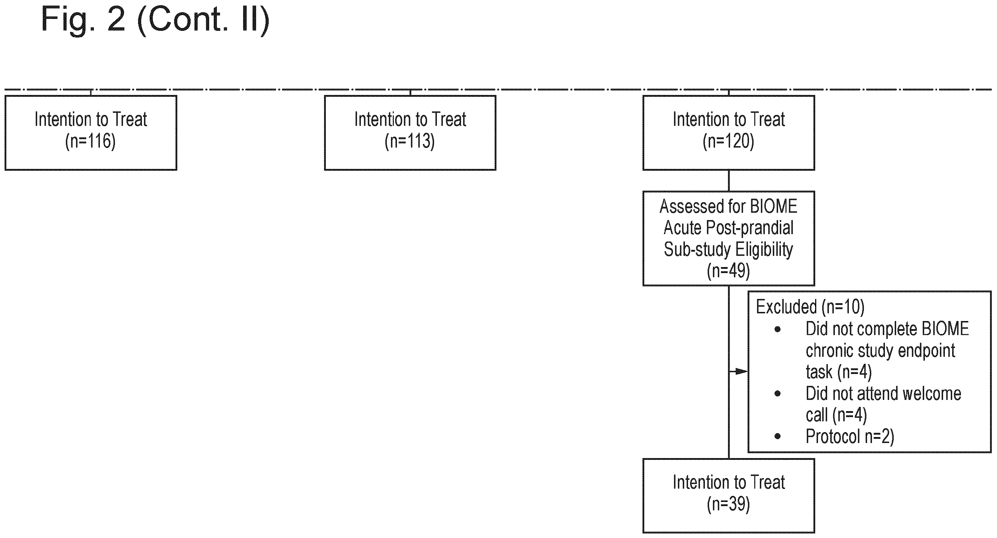

**Figure 2** is a Consolidated Standards of Reporting Trials (CONSORT) diagram summarizing the recruitment of participants to the study, randomisation of participants and reasons for exclusion.

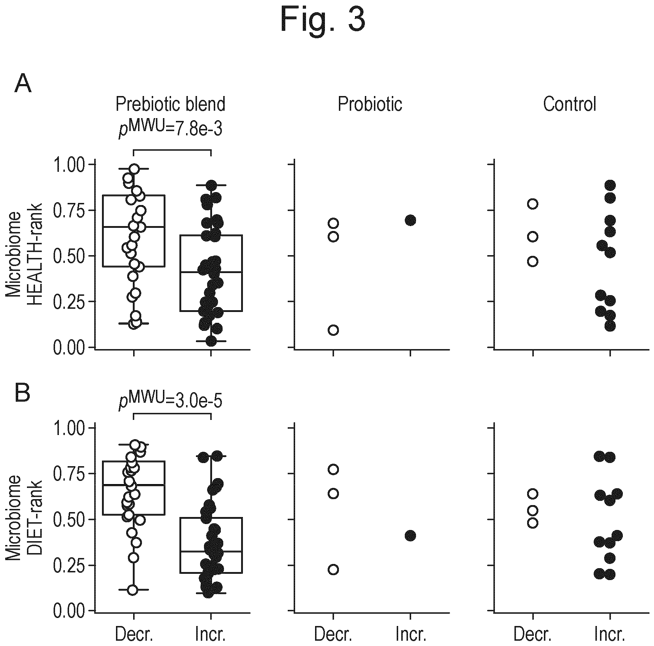

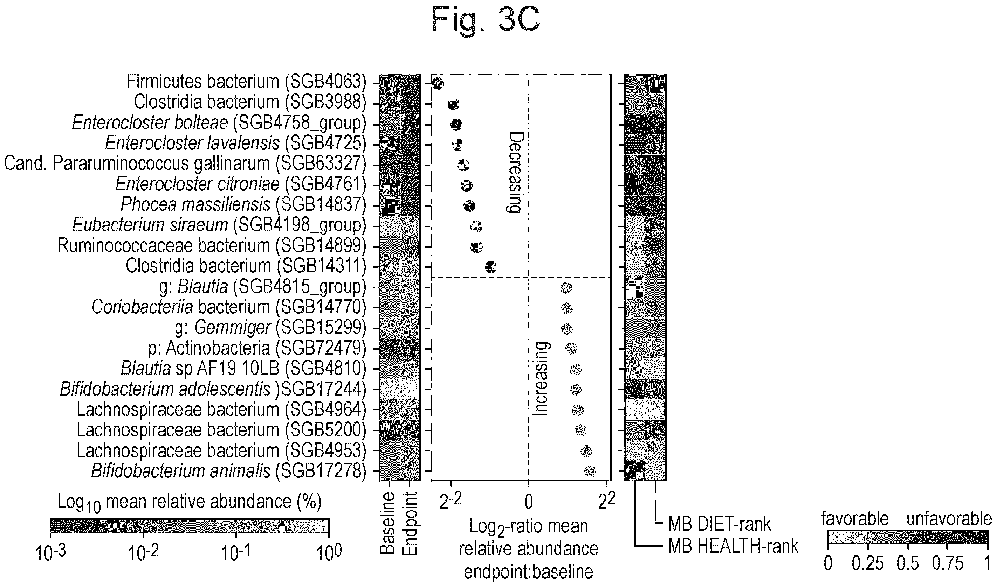

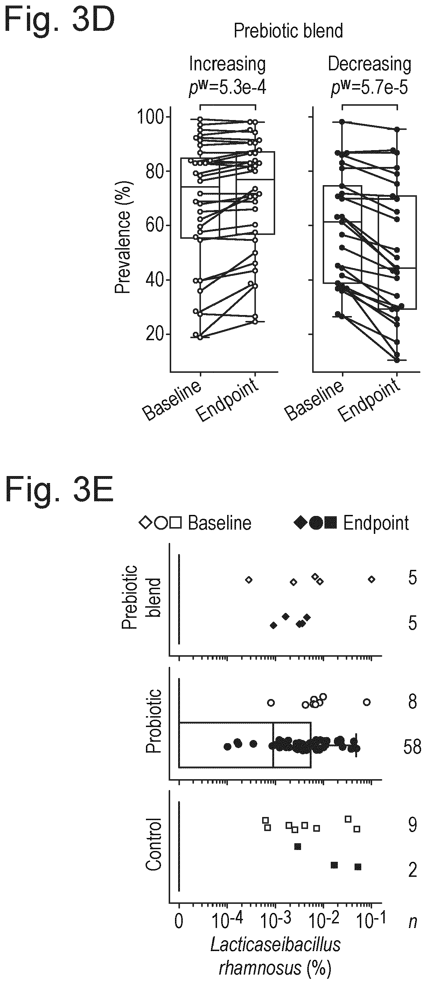

**Figure 3** shows the effect of the prebiotic blend on the gut microbiome. In each group, species with a significant change in their relative abundance at baseline compared to endpoint were identified and categorized into those that decrease or increase at endpoint. For these species, we reported their ZOE MB HEALTH **(****Figure 3a****)** and DIET ranks **(****Figure 3b****)** and only the Prebiotic blend group showed statistically significant differences in the ZOE MB ranks between decreasing and increasing species. This shows that species that decrease at endpoint have higher ZOE MB HEALTH and DIET ranks, meaning these are more unfavourable species. While, on the other hand, species that increase in relative abundance have more favourable ranks. **Figure 3c** shows the top 10 species that increase or increase, according to their log₂-fold change at endpoint and their relative abundance values at both time points, and their change in prevalence. At the end, the ZOE MB HEALTH and DIET ranks are reported. **Figure 3d** shows the prevalence of increasing (left) and decreasing (right) significant species of the prebiotic blend group at baseline and endpoint. Not only these species increase or decrease their relative abundance values, but the favourable species that increase tend also to be more prevalent, i.e., more present across individuals (from 74.1% to 76.9% median prevalence). While the unfavourable species that decrease tend to be much less prevalent among individuals (from 61.3% to 44.3% median prevalence). In **Figure 3E****,** the presence and relative abundance of the probiotic species *Lacticaseibacillus rhamnosus* (formerly known as *Lactobacillus rhamnosis)* was verified among the three groups and only the probiotic group showed a significantly larger number of individuals from which we were able to identify Z. *rhamnosus* in their gut microbiome at 6-weeks. In each section of Figure 3E, the top line represents Baseline and the lower line represents Endpoint.

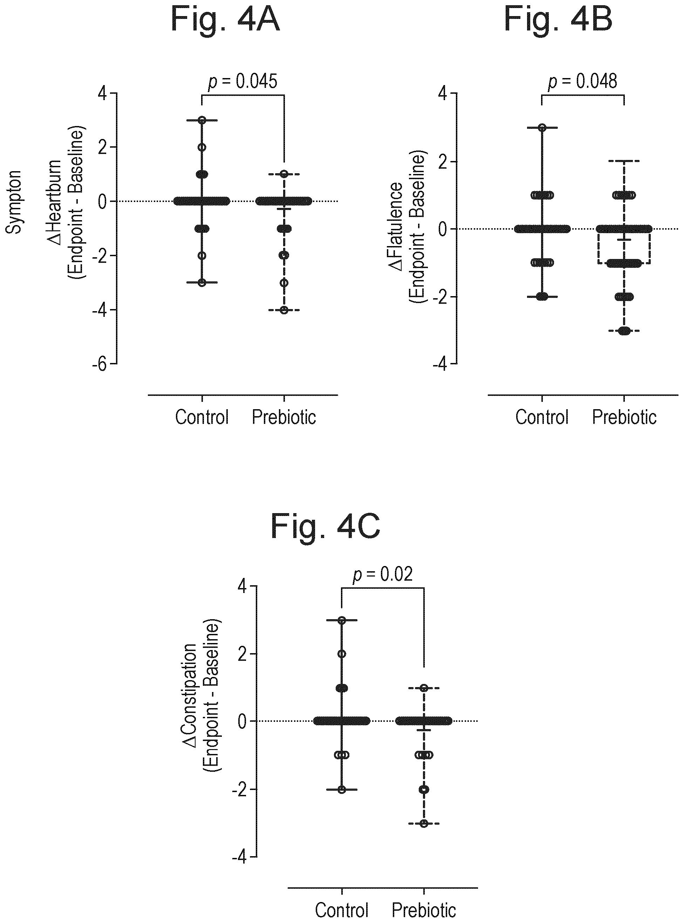

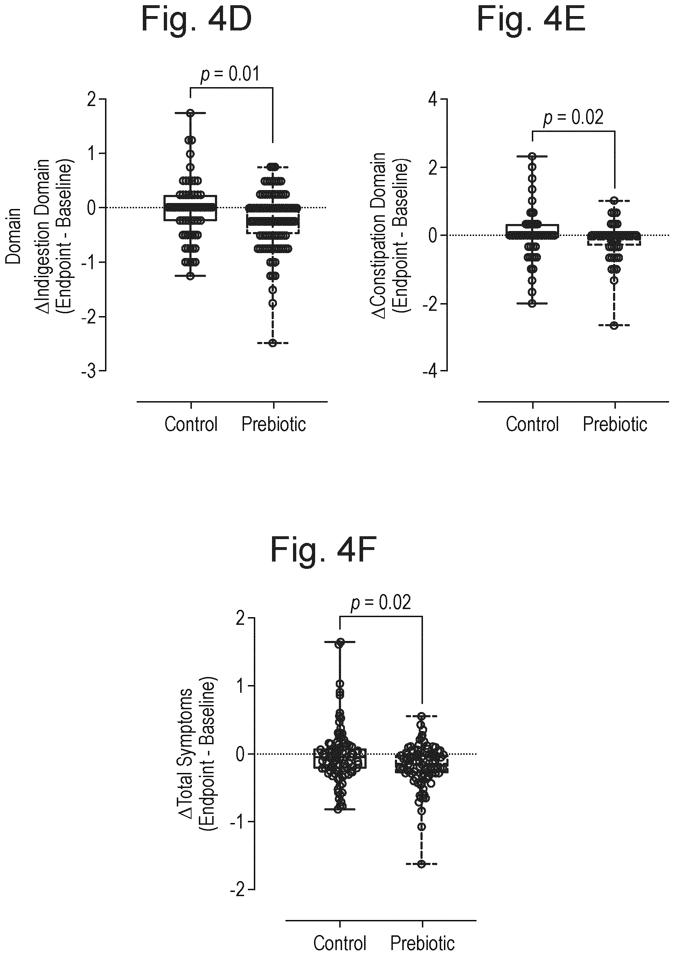

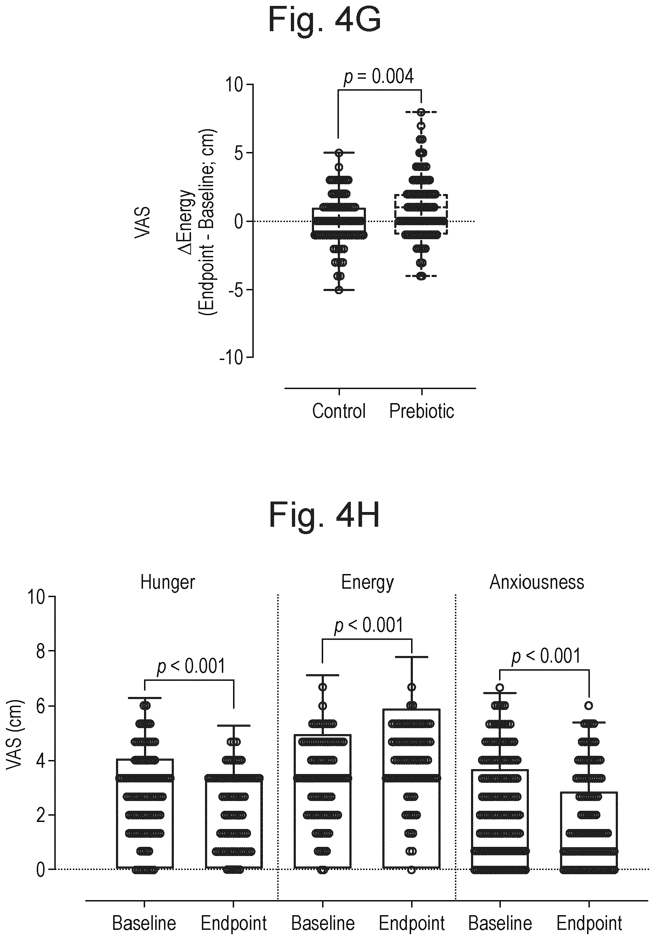

**Figure 4** shows the improvements in selected secondary outcomes in the BIOME study. **Figures 4A-C** show the changes in severity of individual gastrointestinal symptoms including heartburn **(A),** flatulence **(B)** and constipation **(C)** in the prebiotic blend (right-hand box plot) (n = 107) and control (left-hand box plot) (n = 110) groups. **Figures 4D-E** show the changes in severity of gastrointestinal symptom domains including indigestion **(D)** and constipation **(E)** in the prebiotic blend (right-hand box plot) (n = 107) and control (left-hand box plot) (n = 110) groups. **Figure 4F** shows the change in severity of total gastrointestinal symptoms in the prebiotic blend (right-hand box plot) (n = 107) and control (left-hand box plot) (n = 110) groups. **Figure 4G** shows the changes in subjective energy ratings (visual analogue scale, VAS; 0-100mm) from baseline to 6-weeks in the prebiotic blend (right-hand box plot) (n = 107) and control (left-hand box plot) (n = 110) groups. **Figure 4H** shows subjective ratings of hunger, energy and anxiousness in the prebiotic blend group (n = 107) at baseline and endpoint. Data presented are median, first and third quartile (box plots) and range (error bars) for all. The mean value is also presented (+). P-values are the result of Mann-Whitney U-tests **(****Figures 4A-G)** or Wilcoxon signed-rank test **(****Figure 4H****)**

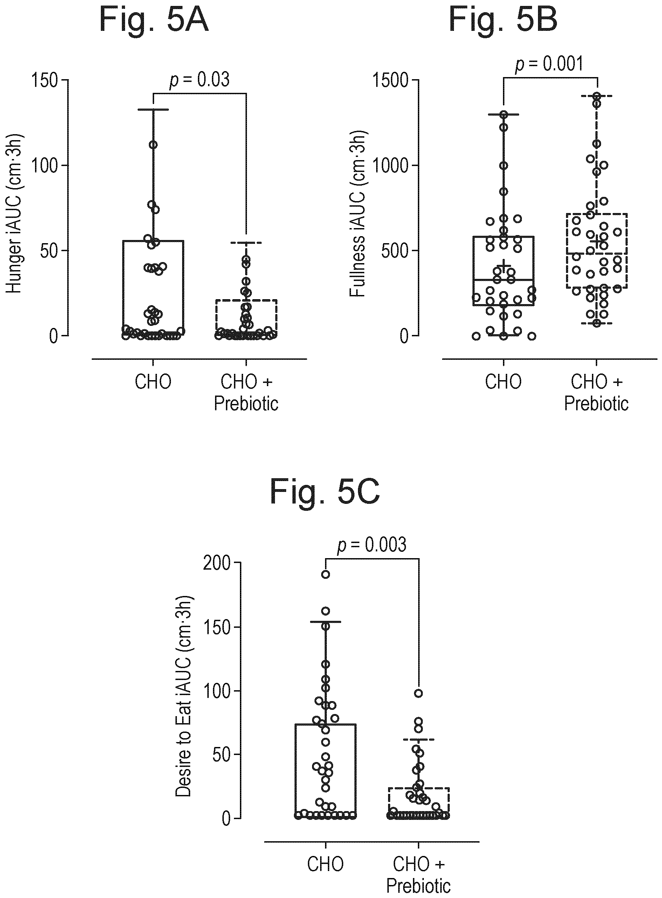

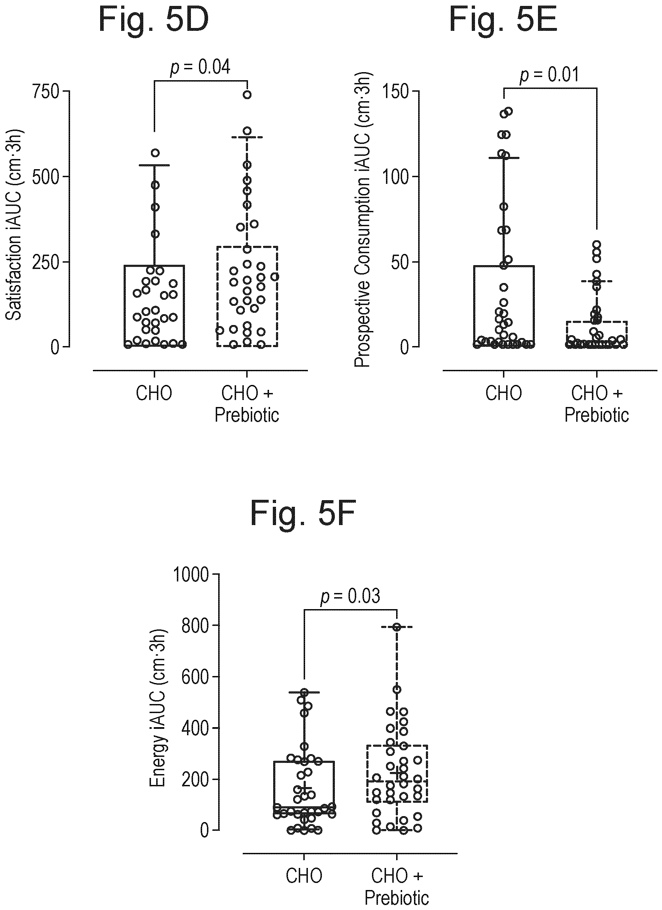

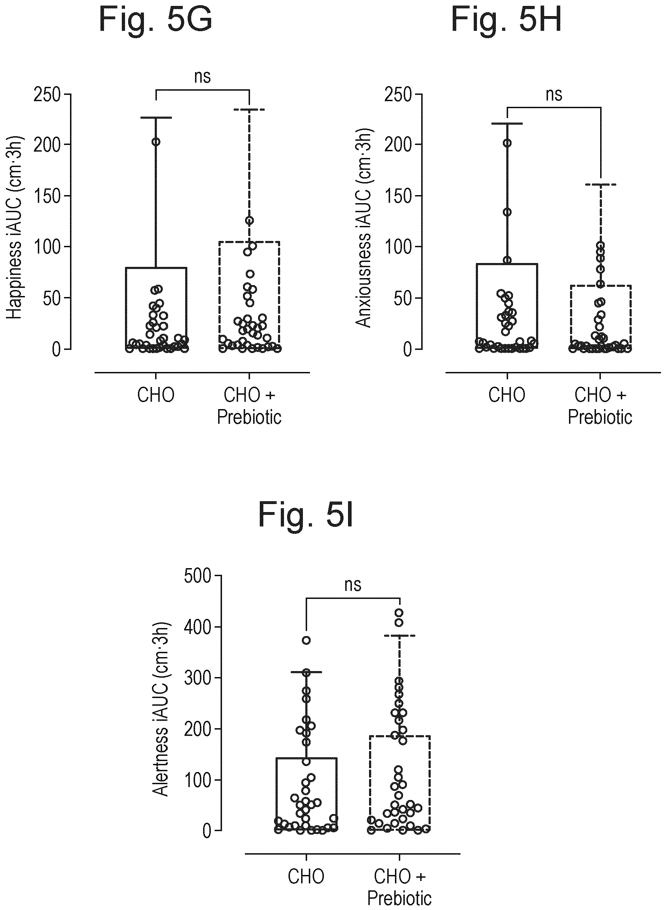

**Figure 5** shows improvements in postprandial subjective ratings of energy, hunger and satiety following consumption of the prebiotic blend in the crossover study (n = 34). Comparison of subject ratings of hunger **(A),** fullness **(B),** desire to eat **(C),** satisfaction **(D),** prospective consumption **(E),** energy **(F),** happiness **(G),** anxiousness **(H),** and alertness **(I)** following consumption of a high carbohydrate meal alone (CHO) or with a prebiotic blend (CHO + prebiotic) (n = 34, crossover design). Ratings were assessed using visual analogue scales completed at 0 hr (immediately before meal consumption, baseline), 15 min, 1hr, 2hr and 3hr. For **Figures 5A****, C, D, E, G-I,** data presented are mean 3h incremental area under the curve (iAUC), standard deviation (box plots) and range (error bars). The mean value is also presented (+). P-values are the result of a linear mixed-effects model with fixed effects for timepoint, meal, and their interaction, and subject ID as a random intercept. For **Figures 5B****, F,** data presented are median 3h incremental area under the curve (iAUC), first and third quartile (box plots) and range (error bars) for all. The mean value is also presented (+). P-values are the result of paired Wilcoxon signed-rank tests.

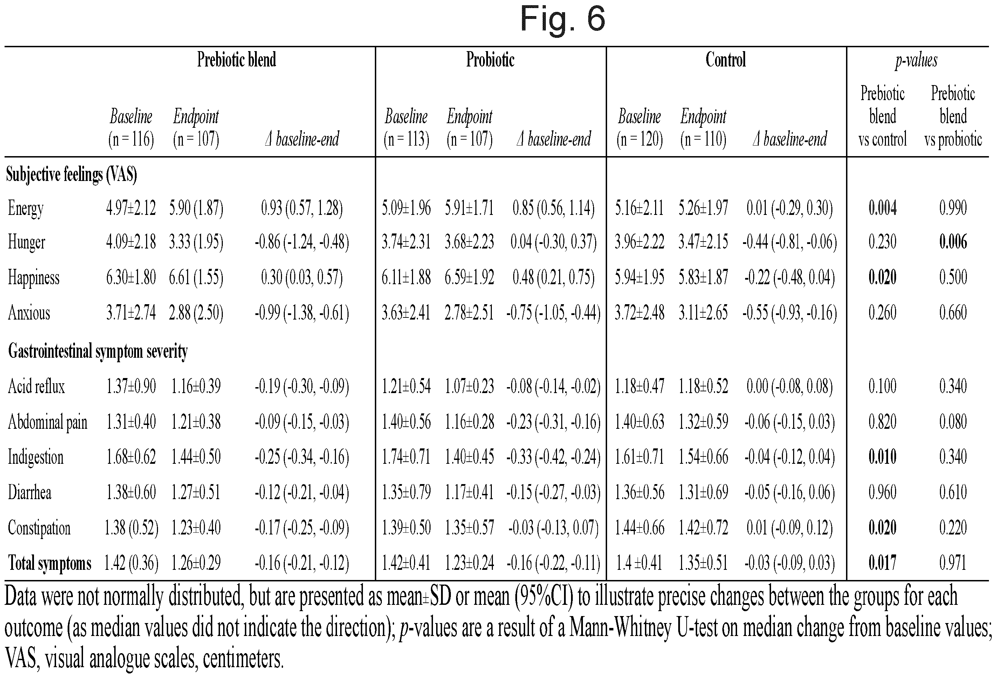

**Figure 6** shows selected secondary outcomes in the BIOME study.

### Detailed Description of the Invention

### Food composition

The invention provides a food composition comprising at least 3 of the following: at least one source of dietary fibre, at least one source of polyphenols, at least one plant protein, at least one source of alpha-linolenic acid (ALA) and at least one source of phytosterols. According to the invention the composition also comprises at least one nut or seed.

Preferably, the food composition comprises at least 4 of the following: at least one source of dietary fibre, at least one source of polyphenols, at least one plant protein, at least one source of alpha-linolenic acid (ALA) and at least one source of phytosterols, and the composition also comprises at least one nut or seed.

More preferably, the food composition comprises at least one source of dietary fibre, at least one source of polyphenols, at least one plant protein, at least one source of alpha-linolenic acid (ALA) and at least one source of phytosterols, and the composition also comprises at least one nut or seed.

As used herein, the term "food composition" refers to a composition which may be ingested by a human or animal. In preferred aspects, the food composition is ingested by a human.

In some aspects, the food composition does not contain a sweetener. As used herein, the term "sweetener" refers to an artificial or natural sweetener, and preferably refers to an artificial sweetener. A sweetener includes, but is not restricted to, sorbitol (E420), mannitol (E421), acesulfame K (E950), aspartame (E951), cyclamic acid and its Na and Ca salts (E952), isomalt (E953), saccharin and its Na, K and Ca salts (E954), sucralose (E955), thaumatin (E957), neohesperidine DC (E959), steviol glycosides (E960), e.g. steviol glycosides from stevia (E960a) and enzymatically produced steviol glycosides (E960c), neotame (E961), salt of aspartame-acesulfame (E962), polyglycitol syrup (E964), maltitol (E965), lactitol (E966), xylitol (E967), and erythritol (E968).

In some aspects, the food composition is savoury. As such, the food composition may have a savoury taste. Preferably, the food composition does not have a sweet taste.

In some aspects, the food composition is suitable for sprinkling on top of a food item. The food item onto which the food composition is sprinkled may be a savoury food item.

In some aspects, the food composition comprises ingredients which have a structural food matrix. As used herein, the term "structural matrix" or "structural food matrix" refers to a complex functional microstructure in food, which affects digestion and absorption of nutrients (i.e. bioaccessibility). In a preferred aspect, the food composition comprises ingredients having their natural structural matrix, i.e. the structural matrix of the natural ingredient has not been mechanically disrupted by milling or powdering. Such a food composition is said to contain ingredients which have a structural matrix that is "maintained". In one aspect, the source of dietary fibre in the food composition disclosed herein has a structural matrix which is maintained. In one aspect, the source of polyphenols in the food composition disclosed herein has a structural matrix which is maintained. In one aspect, the source of alpha linolenic acid (ALA) in the food composition disclosed herein has a structural matrix which is maintained. In one aspect, the source of phytosterols in the food composition disclosed herein has a structural matrix which is maintained. In one aspect, the food composition disclosed herein comprises at least one nut having a structural matrix which is maintained. In one aspect, the food composition disclosed herein comprises at least one seed having a structural matrix which is maintained. In one aspect, the food composition disclosed herein comprises at least two different nuts and at least two different seeds having a structural matrix which is maintained.

In some aspects, the food composition comprises at least one ingredient which is nibbed so as to maintain its structural matrix. In one aspect, the source of dietary fibre in the food composition disclosed herein is nibbed so as to maintain its structural matrix. In one aspect, the source of polyphenols in the food composition disclosed herein is nibbed so as to maintain its structural matrix. In one aspect, the source of alpha linolenic acid (ALA) in the food composition disclosed herein is nibbed so as to maintain its structural matrix. In one aspect, the source of phytosterols in the food composition disclosed herein is nibbed so as to maintain its structural matrix. In a preferred aspect, the food composition disclosed herein comprises at least one nut which is nibbed so as to maintain its structural matrix. In one aspect, the food composition disclosed herein comprises at least one seed which is nibbed so as to maintain its structural matrix. In one aspect, the food composition disclosed herein comprises at least two different nuts and at least two different seeds which nibbed so as to maintain their structural matrix.

In some aspects, the food composition disclosed herein contains ingredients that are not powdered. Powdered ingredients are produced by powdering or milling the natural ingredient. The process of powdering or milling an ingredient mechanically breaks the natural structural matrix of said ingredient. Powdered ingredients therefore do not have a natural structural matrix. In one aspect, the food composition comprises at least 1, at least 2, at least 3, at least 4, at least 5, at least 6, at least 7, at least 8, at least 9, at least 10, at least 11 or at least 12 ingredients that are not powdered. The terms "powdered" and "milled", and "powdering" and "milling", are used interchangeably herein.

In some aspects, the food composition contains ingredients that are present as particles or fragments that are at least 1mm in diameter, for example at least 2mm or at least 5mm in diameter. Inclusion of such larger particles or fragments ensures that a food composition cannot be drunk with water and must be sprinkled on a food item, which encourages maintenance of a healthy diet. In certain embodiments, the food composition comprises at least 1, at least 2, at least 3, at least 4, at least 5, at least 6, at least 7, at least 8, at least 9, at least 10, at least 11 or at least 12 ingredients that are present in particles or fragments that are at least 1m, 2mm or 5mm in diameter. Generally, the larger particles or fragments will be nuts and/or seeds.

In some aspects, the food composition is a wholefood composition. As used herein, the term "whole food" or "wholefood" refers to a food which is free from additives and other artificial substances. Wholefoods are also processed or refined as little as possible. Examples of wholefoods include fruits, vegetables, whole grains and legumes.

In some aspects, the food composition does not contain an anti-caking agent. Anti-caking agents may include, but are not limited to, calcium stearate, magnesium stearate, sodium bicarbonate, sodium ferrocyanide, potassium ferrocyanide, 538 calcium ferrocyanide, calcium phosphate, sodium silicate, silicon dioxide, calcium silicate, magnesium trisilicate, talcum powder, sodium aluminosilicate, potassium aluminium silicate, calcium aluminosilicate, bentonite, aluminium silicate, stearic acid, polydimethylsiloxane. In one aspect, the food composition does not contain silicon dioxide.

In some aspects, the food composition does not contain an artificial or cosmetic additive. Additives are substances which are added to food to enhance its flavour or appearance, or to preserve it. Artificial flavours, flavour enhancers, colours, emulsifiers, emulsifying salts, sweeteners, thickeners, and anti-foaming, bulking, carbonating, foaming, gelling and glazing agents are all examples of additives. Additives may include, but are not limited to, lecithins, calcium phosphate, zinc citrate, coenzyme Q10, nicotinic acid, nicotinamide, riboflavin, beta carotene, pyridoxine hydrochloride, manganese amino acid chelate, copper gluconate, folate, chromium picolinate, biotin, menaquinone-7, vitamins (Vitamin C (as L-Ascorbic Acid), Vitamin K (K2, as Menaquinone-7), Vitamin A (as Retinyl Acetate), Vitamin E (D-Alpha Tocopherol Acetate), Niacin (as Niacinamide), Vitamin B 12 (as Cyanocobalamin), Vitamin D (D2 as Ergocalciferol, D3 as Plant-Derived Cholecalciferol), Pantothenic Acid (as Calcium D-Pantothenate), Vitamin B6 (as Pyridoxine Hydrochloride), Vitamin B2 (as Riboflavin), Folate (as Calcium-L-Methylfolate), Vitamin B1 (as Thiamin Mononitrate)) and Lutein). In one aspect, the food composition does not contain artificial stabilisers. Artificial stabilisers may include, but are not limited to, Guar Gum, Xanthan Gum, Faba Bean Protein.

In some aspects, the food composition comprises at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31 or at least 32 different ingredients selected from the list consisting of flaxseed, chia seeds, sunflower seeds, pumpkin seeds, hemp seeds, red lentil flakes, grape seed, almonds, hazelnuts, walnuts, chicory root inulin fibre, puffed quinoa, white mushroom, thyme, onion, parsley, turmeric, cumin, Lion's Mane mushroom, Reishi mushroom, Chaga mushroom, Shiitake mushroom, Cordyceps mushroom, Maitake mushroom, Tremella mushroom, rosemary, garlic, red beetroot flakes, carrot flakes, nutritional yeast flakes, baobab powder, and buckthorn. In some aspects, the food composition comprises ingredients from at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24 or at least 25 different plants. Preferably, the composition comprises at least 3, at least 4, or at least 5 sources of dietary fibre, at least 3, at least 4, or at least 5 sources of polyphenols, at least 3, at least 4, or at least 5 sources of plant protein, at least 3, at least 4, or at least 5 sources of ALA, and at least 3, at least 4, or at least 5 sources of phytosterols.

In certain embodiments, the food composition comprises at least three, four or all five of:
(a) at least two, three or four sources of dietary fibre selected from the group consisting of flaxseed, chia seed, sunflower seeds, red lentil flakes, chicory root inulin fibre, nutritional yeast flakes, almonds, hazelnuts, walnuts, brazil nuts, puffed quinoa, puffed buckwheat and red beetroot flakes;
(b) at least two, three or four sources of polyphenols selected from the group consisting of mushroom powder, thyme, onion powder, parsley, rosemary, turmeric and cumin;
(c) at least one of quinoa and buckwheat;
(d) at least one of flaxseed and chia seed; and
(e) at least two, three or four sources of phytosterols selected from the list consisting of sunflower seeds, pumpkin seeds, hemp seed, almonds, hazelnuts, walnuts, and brazil nuts.

In preferred embodiments, the food composition comprises:
(a) at least six sources of dietary fibre selected from the group consisting of flaxseed, chia seed, sunflower seeds, red lentil flakes, chicory root inulin fibre, nutritional yeast flakes, almonds, hazelnuts, walnuts, brazil nuts, puffed quinoa, puffed buckwheat and red beetroot flakes;
(b) at least four sources of polyphenols selected from the group consisting of mushroom powder, thyme, onion powder, parsley, rosemary, turmeric and cumin;
(c) at least one of quinoa and buckwheat;
(d) at least one of flaxseed and chia seed; and
(e) at least four sources of phytosterols selected from the list consisting of sunflower seeds, pumpkin seeds, hemp seed, almonds, hazelnuts, walnuts, and brazil nuts,
and at least five ingredients are present in particles or fragments that are at least 1m, 2mm or 5mm in diameter.

In one aspect, the food composition is gluten free. In another aspect, the food composition is vegetarian. In another aspect, the food composition is vegan. In another aspect, the food composition is dairy-free. In another aspect, the food composition does not contain added sugar. In another aspect, the food composition has a shelf life of at least 9 months.

In certain embodiments, the food composition does not comprise any bacterial strains. In certain embodiments, the food composition is not a probiotic composition. Such compositions may comprise de minimis amounts of bacteria, in that they are not sterile, but they will not contain significant amounts of any bacteria and will not have cultures of bacteria added to them. Prebiotic compositions that do not contain bacterial strains may be suitable for a wider range of subjects, because probiotic strains are often suitable only for subjects that are deficient in the relevant strain, and probiotic strains may be unsuitable for subjects with different microbiome profiles.

In a particularly preferred embodiment, the food composition comprises flaxseed, chia seeds, sunflower seeds, pumpkin seeds, hemp seeds, red lentil flakes, grape seed, almonds, hazelnuts, walnuts, chicory root inulin fibre, puffed quinoa, white mushroom, thyme, onion, parsley, turmeric, cumin, Lion's Mane mushroom, Reishi mushroom, Chaga mushroom, Shiitake mushroom, Cordyceps mushroom, Maitake mushroom, Tremella mushroom, rosemary, garlic, red beetroot flakes, carrot flakes, nutritional yeast flakes, baobab powder, and buckthorn, and the food composition is suitable for sprinkling on top of a food item, wherein at least one of the nuts is nibbed so as to maintain its structural matrix.

In a particularly preferred embodiment, the food composition consists of flaxseed, chia seeds, sunflower seeds, pumpkin seeds, hemp seeds, red lentil flakes, grape seed, almonds, hazelnuts, walnuts, chicory root inulin fibre, puffed quinoa, white mushroom, thyme, onion, parsley, turmeric, cumin, Lion's Mane mushroom, Reishi mushroom, Chaga mushroom, Shiitake mushroom, Cordyceps mushroom, Maitake mushroom, Tremella mushroom, rosemary, garlic, red beetroot flakes, carrot flakes, nutritional yeast flakes, baobab powder, and buckthorn, and the food composition is suitable for sprinkling on top of a food item, wherein at least one of the nuts is nibbed so as to maintain its structural matrix.

### Dietary fibre

According to the invention, the food composition comprises at least one source of dietary fibre. In one aspect, the food composition comprises at least 2, at least 3, at least 4, at least 5, at least 6, at least 7, at least 8, at least 9 or at least 10 sources of dietary fibre.

The food composition disclosed herein may comprise at least 1%, at least 5%, at least 10%, at least 15%, at least 20%, at least 25% or at least 30% dietary fibre.

The source of dietary fibre may be obtained from one or more of: flaxseed, chia seed, sunflower seeds, red lentil flakes, chicory root inulin fibre, nutritional yeast flakes, almonds, hazelnuts, walnuts, brazil nuts, puffed quinoa, puffed buckwheat and red beetroot flakes.

The source of dietary fibre may be obtained from one or more plants. The source of dietary fibre may obtained from one or more seeds. The source of dietary fibre may be flaxseed. Acute flaxseed intake has been shown to reduce glucose levels after meals in people with type 2 diabetes (Moreira et al. Nutrients. 2022; 14(18):3736). The source of dietary fibre may be chia seeds. The source of dietary fibre may be sunflower seeds.

The source of dietary fibre may be red lentil flakes. Red lentils are a rich source of fibre and there is some evidence to suggest that they may be able to improve blood sugar responses after meals (Clarke et al. Nutrients. 2022;14(4):849).

The source of dietary fibre may be chicory root fibre (inulin). Chicory root fibre (inulin) is a source of fibre, and is regarded to have prebiotic effects on gut microbiota, promoting the abundances of certain bacteria (Bifidobacterium, Lactobacillus, and Faecalibacterium prausnitzii) (Hughes et al. Adv Nutr. 2022;13(2):492-529).

The source of dietary fibre may be nutritional yeast flakes.

The source of dietary fibre may be beetroot. Preferably, the source of dietary fibre may be beetroot flakes. The source of dietary fibre may be beetroot powder. Beetroot powder has a higher total antioxidant potential than juice, chips, or cooked beetroot.

The source of dietary fibre may be one or more nuts. The source of dietary fibre may be one or more tree nuts. The source of dietary fibre may be almonds. The source of dietary fibre may be hazelnuts. The source of dietary fibre may be walnuts. The source of dietary fibre may be brazil nuts.

The source of dietary fibre may be quinoa. Preferably, the source of dietary fibre is puffed quinoa.

The source of dietary fibre may be buckwheat. Preferably, the source of dietary fibre is puffed buckwheat.

### Polyphenols

Polyphenols are naturally occurring compounds typically found in plants. They are useful for protection against oxidative stress and may play a role in the prevention of chronic diet-related diseases, coronary heart disease and inflammation. Beneficial polyphenols include, but are not limited to, p-coumaric acid, protocatechuic acid, ferulic acid, vanillic acid, p-hydroxybenzoic acid and syringic acid.

According to the invention, the food composition comprises at least one source of polyphenol. In one aspect, the food composition comprises at least 2 sources of polyphenol. In another aspect, the food composition comprises at least 3 sources of polyphenol. In another aspect, the food composition comprises at least 4 sources of polyphenol. In another aspect, the food composition comprises at least 5 sources of polyphenol. In another aspect, the food composition comprises at least 6 sources of polyphenol. In another aspect, the food composition comprises at least 7 sources of polyphenol.

The source of polyphenol may be obtained from one or more spices. Preferably, the source of polyphenol is obtained from one or more of the following: mushroom powder, thyme, onion powder, parsley, rosemary, turmeric and cumin. Mushroom powder may comprise one or more of the following: Lions Mane, Reishi, Chaga, Shiitake, Cordyceps, Maitake, Tremella. Studies using spice/herb mixes that contain similar ingredients have suggested that increased intakes are associated with modulations in gut microbiota composition, and some spice/herb studies have shown associations with favourable effects on other markers of health, such as blood pressure and inflammation (in adults at risk of cardiometabolic diseases) (Petersen et al. Am J Clin Nutr. 2021;114(6):1936-1948; Oh et al. Am J Clin Nutr. 2022;115(1):61-72). Spice mixes have also been shown to alter gut microflora composition (Khine et al. Sci Rep (2021)11, 11264).

The source of polyphenol may be obtained from one or more of the following: flaxseed, baobab powder (such as baobab fruit pulp powder), grape seed powder, garlic, buckthorn powder, red beetroot flakes, and carrot flakes. Research suggests that chlorogenic acid (which can be found in carrot flakes) can play an important role in regulating glycolipids metabolism (Yu et al. Asia Pac J Clin Nutr. 2022;31(4):602-610).

### Plant protein

According to the invention, the food composition comprises at least one plant protein. Many of the other ingredients of the food composition disclosed herein will contain plant proteins, because all plants contain at least some plant protein.

The plant protein may be obtained from one or more of wholegrains and legumes. Wholegrain intake has been associated with several benefits to human health, including reduced risk of cardiovascular disease and cancer (Aune et al. BMJ 2016; 353).

The plant protein may be obtained from one or more of quinoa and buckwheat. Preferably, the plant protein is obtained from one or more of puffed quinoa and puffed buckwheat.

### Alpha linolenic acid (ALA)

ALA is an important omega-3 fatty acid to consume from our diet because it is not synthesised by the human body (i.e. it is an essential fatty acid). High intakes of ALAs have been associated with a reduced risk of coronary heart disease (Wei et al. British Journal of Nutrition. 2018;119(1):83-89). Nuts and seeds are a good source of ALAs. Research also suggests there is a probable relationship between nut and seed consumption and lower cardiovascular disease risk (Arnesen et al. Food Nutr Res. 2023;67:10.29219).

According to the invention, the food composition comprises at least one source of ALA. In one aspect, the food composition comprises at least 2 sources of ALA. In another aspect, the food composition comprises at least 3 sources of ALA.

The source of ALA may be obtained from one or more seeds.

The source of ALA may be flaxseed. Flaxseed is a rich source of fibre, lignans (a polyphenol) and ALA. There is some evidence to show that flaxseed may reduce blood pressure and lower cholesterol levels, and it has therefore been associated with beneficial effects on cardiovascular disease risk. High intakes have also been linked with reduced blood sugar spikes after meals in individuals with type 2 diabetes.

The source of ALA may be chia seeds. Chia seeds are a rich source of fibre and omega 3s (ALA) (Silva et al. FoodFunct. 2021;12(19):8835-8849). Evidence suggests that chia seeds may have a protective effect on blood fat profiles and improve cholesterol levels (Enes et al. J Food Sei. 2020;85(2):226-239). Some evidence has also suggested that chia seeds may be associated with improved blood glucose control (Teoh et al. Nutr Rev. 2018;76(4):219-242).

The source of ALA may also be hemp seeds.

In some aspects, the food composition comprises at least one source of omega-3 fatty acids. The three main omega-3 fatty acids are alpha-linolenic acid (ALA), eicosapentaenoic acid (EPA), and docosahexaenoic acid (DHA). In some aspects, the food composition comprises at least one of alpha-linolenic acid (ALA), eicosapentaenoic acid (EPA), and docosahexaenoic acid (DHA).

In some aspects, the food composition comprises at least one source of polyunsaturated fatty acids (PUFAs). Replacing saturated fat with PUFAs, MUFAs (monounsaturated fatty acids), or high quality carbohydrates is associated with lower coronary heart disease events (Clifton and Keogh Nutr Metab Cardiovasc Dis. (2017) 27(12): 1060-1080).

### Phytosterols

Almonds, which contain a high proportion of phytosterols, can benefit gut health by influencing gut microbiome diversity and the production of short chain fatty acids in the gut, such as butyrate. Therefore, other sources of phytosterols may be able to benefit gut health in a similar way.

According to the invention, the food composition comprises at least one source of phytosterol. In one aspect, the food composition comprises at least 2 sources of phytosterol. In another aspect, the food composition comprises at least 3 sources of phytosterol. In another aspect, the food composition comprises at least 4 sources of phytosterol. In another aspect, the food composition comprises at least 5 sources of phytosterol. In another aspect, the food composition comprises at least 6 sources of phytosterol. In another aspect, the food composition comprises at least 7 sources of phytosterol.

The source of phytosterols may be obtained from one or more seeds. The source of phytosterols may be obtained from one or more nuts. The source of phytosterols may be obtained from one or more tree nuts.

The source of phytosterols may be one or more of the following: sunflower seeds, pumpkin seeds, hemp seed, almonds, hazelnuts, walnuts, and brazil nuts.

### Methods

The invention provides a method of supplementing the dietary needs of a human, comprising administering to the human the food composition disclosed herein. The invention provides a food composition disclosed herein for use in a method of supplementing the dietary needs of a human.

The invention also provides a method of benefitting the gut microbiome of a human using the food composition disclosed herein. The invention provides a food composition disclosed herein for use in a method of benefitting the gut microbiome of a human.

In certain embodiments, the invention provides a method of increasing the abundance of one or more pro-health indicator microbes in the gut microbiome of a human using the food composition disclosed herein. In certain embodiments, the pro-health indicator microbes are selected from the group consisting of Prevotella copri, Blastocystis spp., Haemophilus parainfluenzae, Firmicutes bacterium CAG 95, Bifidobacterium animalis, Oscillibacter sp 57 20, Roseburia sp CAG 182, Veillonella dispar, Eubacterium eligens, Firmicutes bacterium CAG 170, Rothia mucilaginosa, Veillonella infantium, Roseburia hominis, Oscillibacter sp PC13, Clostridium sp CAG 167, Ruminococcaceae bacterium D5, Paraprevotella xylaniphila, Faecalibacterium prausnitzii, Romboutsia ilealis, and Veillonella atypica. In other certain embodiments, the pro-health indicator microbes (defined by SGB designation) are selected from the group consisting of SGB 15249, SGB6340, SGB4964, SGB14252, SGB15229, SGB6174 group, SGB15317, SGB14179, SGB15225, SGB4894, SGB4643, SGB4963, SGB79840, SGB4893, SGB6276, SGB3952, SGB4638, SGB15236, SGB4191, SGB15053 group, SGB15368, SGB4782, SGB14042, SGB4706, SGB4644, SGB49188, SGB4781, SGB4777, SGB14921, SGB15234, SGB8601, SGB5087, SGB14311, SGB4953, SGB7258, SGB4882, SGB6367, SGB15106, SGB4778, SGB15131, SGB4198 group, SGB15031, SGB13981, SGB15123, SGB54300, SGB4665, SGB13979, SGB15410, SGB2290, SGB14954, SGB14306, SGB4805, SGB14899, SGB4803, SGB13982, SGB15265 group, SGB14114, SGB47656, SGB6749, SGB14253, SGB15346, SGB4810, SGB4770, SGB25497, SGB4957, SGB4654, SGB15373, SGB15254, SGB15323, SGB71759, SGB15180, SGB49168, SGB15051, SGB15145, SGB4966, SGB4780, SGB15291, SGB4816, and SGB4714.

In certain embodiments, the invention provides a method of decreasing the abundance of one or more poor-health indicator microbes in the gut microbiome of a human using the food composition disclosed herein. In certain embodiments, the poor-health indicator microbes are selected from the group consisting of Eubacterium ventriosum, Roseburia inulinivorans, Clostridium spiroforme, Clostridium bolteae CAG 59, Eggerthella lenta, Clostridium bolteae, Collinsella intestinalis, Clostridium innocuum, Blautia obeum, Clostridium symbiosum, Clostridium sp CAG 58, Blautia hydrogenotrophica, Anaerotruncus coli hominis, Ruminococcus gnavus, Flavonifractor plautii, Clostridium leptum, Ruthenibacterium lactatiformans, and Escherichia coli. In other certain embodiments, the poor health indicator microbes (defined by SGB designation) are selected from the group consisting of SGB7253, SGB6769, SGB4721, SGB14837, SGB14546 group, SGB4763, SGB5193, SGB7985, SGB4699, SGB19850 group, SGB17137, SGB4785, SGB15078, SGB53821, SGB15452, SGB15271, SGB4724, SGB8255 group, SGB79823, SGB8028 group, SGB4573 group, SGB14874, SGB4988, SGB5184, SGB4786, SGB4826 group, SGB4041, SGB7984, SGB4761, SGB4447, SGB6744, SGB1836 group, SGB1814, SGB4630 group, SGB8163, SGB4617, SGB4588 group, SGB4742, SGB4572, SGB10115, SGB15158, SGB29328, SGB4791, SGB4688, SGB10068, SGB71883, SGB4760, SGB4037 group, SGB4529, SGB4837 group, SGB79883, SGB4762, SGB4797, SGB14809, SGB4758 group, SGB4703, SGB4606, SGB4584, SGB15132, SGB4746, SGB4862, SGB4798, SGB4861, SGB4608, SGB4035, SGB4794 group, SGB4753, and SGB4583.In the preceding paragraphs, some of the microbes are primarily identified by a Species-level genome bin (SGB) number designation, e.g., SGB6340. Species-level genome bins are clusters of similar genomes, described by Segata Lab of Computational Metagenomics, University of Trento (available online at segatalab.cibio.unitn.it/data/Pasolli_et_al.html). Some SGBs correspond 1-1 with a known species. Some known species sub-divide into multiple SGBs. Some SGBs are for species which are not yet known.

SGBs are defined in Pasolli et al. (Cell 176(3):P659-662.E20, 2019; doi.org/10.1016/j.cell.2019.01.001). In brief, all genomes are organized into clusters (SGBs) based on their full-genome genomic distance (ANI, >=95%). SGB clusters with a reference genomes are considered as 'known' (kSGBs) and SGB clusters of only computationally reconstructed genomes are considered as 'unknown' (uSGBs). SGBs are further clustered into genus-level genome bins (GGBs) and family-level genomes bins (FGBs) based on ANI >=85% and >=70%, respectively. This is also exploited to consider taxonomic labels of known genomes down-to either the genus or family level and propagated to all SGBs clustered in the same GGB or FGB cluster.

SGBs can be profiled using MetaPhlAn 4 (Metagenomic Phylogenetic Analysis; Blanco-Miguez et al., Nat Biotechnol, 2023, doi.org/10.1038/s41587-023-01688-w) that uses in its database some small pieces of unique genomic sequences that allows for the accurate identifications of the SGBs. The MetaPhlAn version used to profile microbiome samples described herein is "4.beta.3"; the SGBs database version used is "vJan21_CHOCOPhlAnSGB_202103".

In some instances, including sometimes under the strict threshold of 95% Average Nucleotide Identity (ANI), there can be a large SGB cluster surrounded by multiple small SGB clusters. In this circumstance, definition unique DNA piece(s) to allow their identifications may be difficult as the task of identifying these unique DNA pieces is hampered by the presence of these small SGB clusters. Hence, for these instances, the large SGB cluster is labeled as "group" and the closets smaller SGB clusters will be considered together with the big SGB cluster when searching for the unique DNA pieces.

In some instances, the pro-health indicator microbes are selected from Table 1B and the poor health indicator microbes are selected from Table 1C. The tables below provide lists of microbes including the "Good Bug" SGBs and "Bad Bug" SGBs. Table 1A is a ranked list of 661 microbes as evaluated by health alone and by diet alone. Tables 1B and 1C provide the union of the top / bottom microbes from the microbe rankings as evaluated by health with and without diet; Table 1B shows the 79 collective "GOOD BUG" SGBs, and Table 1C shows the 68 collective "BAD BUG" SGBs. These tables show the respective rank of each listed microbe, along with its identification based on SGB designation (first column); the "group" designation is discussed above. "Known / Unknown" indicates whether the assigned SGB is defined as 'known' (k) or 'unknown' (u); this reflects whether such a defined cluster represents an already known species or it is only represented by computationally reconstructed genomes. The "Level taxonomy" column reports the level to which the taxonomy is assigned. For kSGB this will always be "Species" because (being known) there is a reference genome with a species taxonomic label assigned to it. For categorization "uSGB", the taxonomy level instead can be one of "Genus", "Family", or "Other"; this represents how close a known SGB is to that uSGB. The final column "species label" reports an informal way to quickly identify the SGBs, which may or may not correspond to a previously recognized taxonomic species.

**Table 1A - Identification and Ranking of Spectrum of Good, Bad, and Neutral microbes**

| **SGB** | **Health Rank** | **Diet Rank** | **SGB** | **Health Rank** | **Diet Rank** |
|---|---|---|---|---|---|
| **SGB15249** | 0.013 | 0.218 | **SGB6140** | 0.520 | 0.638 |
| **SGB6340** | 0.014 | 0.094 | **SGB14127** | 0.521 | 0.893 |
| **SGB4964** | 0.036 | 0.095 | **SGB29339** | 0.524 | 0.752 |
| **SGB14252** | 0.047 | 0.233 | **SGB1832** | 0.524 | 0.315 |
| **SGB15229** | 0.052 | 0.281 | **SGB9342_group** | 0.524 | 0.619 |
| **SGB6174_group** | 0.057 | 0.247 | **SGB15278** | 0.528 | 0.413 |
| **SGB15317** | 0.059 | 0.188 | **SGB9203** | 0.528 | 0.395 |
| **SGB14179** | 0.071 | 0.290 | **SGB1962** | 0.529 | 0.391 |
| **SGB15225** | 0.072 | 0.169 | **SGB5076** | 0.531 | 0.315 |
| **SGB4894** | 0.072 | 0.197 | **SGB4808** | 0.532 | 0.227 |
| **SGB4643** | 0.072 | 0.142 | **SGB15119** | 0.532 | 0.157 |
| **SGB4963** | 0.073 | 0.145 | **SGB3962** | 0.535 | 0.795 |
| **SGB79840** | 0.073 | 0.180 | **SGB14892** | 0.536 | 0.778 |
| **SGB4893** | 0.075 | 0.126 | **SGB4775** | 0.536 | 0.837 |
| **SGB6276** | 0.079 | 0.280 | **SGB4166** | 0.537 | 0.534 |
| **SGB3952** | 0.089 | 0.375 | **SGB7144** | 0.537 | 0.532 |
| **SGB4638** | 0.089 | 0.280 | **SGB4959** | 0.538 | 0.562 |
| **SGB15236** | 0.093 | 0.246 | **SGB63353** | 0.539 | 0.640 |
| **SGB4191** | 0.094 | 0.227 | **SGB14953** | 0.542 | 0.668 |
| **SGB15053_group** | 0.099 | 0.180 | **SGB6139** | 0.545 | 0.447 |
| **SGB15368** | 0.101 | 0.235 | **SGB16971** | 0.545 | 0.295 |
| **SGB4782** | 0.103 | 0.127 | **SGB15300** | 0.545 | 0.463 |
| **SGB14042** | 0.103 | 0.196 | **SGB3574** | 0.545 | 0.847 |
| **SGB4706** | 0.109 | 0.084 | **SGB47850** | 0.545 | 0.143 |
| **SGB4644** | 0.116 | 0.262 | **SGB63327** | 0.545 | 0.896 |
| **SGB49188** | 0.116 | 0.445 | **SGB4181** | 0.547 | 0.652 |
| **SGB4781** | 0.119 | 0.200 | **SGB15506** | 0.547 | 0.537 |
| **SGB4777** | 0.119 | 0.056 | **SGB5190** | 0.548 | 0.272 |
| **SGB14921** | 0.121 | 0.285 | **SGB14181** | 0.548 | 0.605 |
| **SGB15234** | 0.124 | 0.404 | **SGB1846** | 0.548 | 0.435 |
| **SGB8601** | 0.128 | 0.201 | **SGB15068** | 0.549 | 0.432 |
| **SGB5087** | 0.129 | 0.184 | **SGB4537** | 0.550 | 0.367 |
| **SGB14311** | 0.131 | 0.493 | **SGB14906** | 0.551 | 0.466 |
| **SGB4953** | 0.132 | 0.229 | **SGB9347** | 0.551 | 0.315 |
| **SGB7258** | 0.132 | 0.116 | **SGB1786** | 0.551 | 0.433 |
| **SGB4882** | 0.132 | 0.152 | **SGB7265** | 0.553 | 0.776 |
| **SGB6367** | 0.133 | 0.163 | **SGB4571** | 0.556 | 0.375 |
| **SGB15106** | 0.134 | 0.243 | **SGB29321** | 0.557 | 0.599 |
| **SGB4778** | 0.140 | 0.106 | **SGB4531** | 0.557 | 0.824 |
| **SGB15131** | 0.142 | 0.461 | **SGB15073** | 0.560 | 0.372 |
| **SGB4198_group** | 0.144 | 0.585 | **SGB14933** | 0.562 | 0.424 |
| **SGB15031** | 0.150 | 0.428 | **SGB15154** | 0.564 | 0.549 |
| **SGB13981** | 0.154 | 0.220 | **SGB6747** | 0.565 | 0.794 |
| **SGB15123** | 0.162 | 0.282 | **SGB15273** | 0.568 | 0.723 |
| **SGB54300** | 0.164 | 0.397 | **SGB4367** | 0.569 | 0.530 |
| **SGB4665** | 0.166 | 0.138 | **SGB15091** | 0.572 | 0.431 |
| **SGB13979** | 0.166 | 0.216 | **SGB3965** | 0.573 | 0.251 |
| **SGB15410** | 0.169 | 0.215 | **SGB63369** | 0.574 | 0.666 |
| **SGB2290** | 0.169 | 0.314 | **SGB9224** | 0.575 | 0.648 |
| **SGB14954** | 0.170 | 0.274 | **SGB3964** | 0.575 | 0.618 |
| **SGB14306** | 0.173 | 0.263 | **SGB6952** | 0.580 | 0.503 |
| **SGB4805** | 0.173 | 0.604 | **SGB4765** | 0.581 | 0.270 |
| **SGB14899** | 0.174 | 0.759 | **SGB29305** | 0.581 | 0.640 |
| **SGB4803** | 0.174 | 0.491 | **SGB4285_group** | 0.581 | 0.627 |
| **SGB13982** | 0.177 | 0.364 | **SGB5089** | 0.584 | 0.400 |
| **SGB15265_group** | 0.177 | 0.289 | **SGB4581** | 0.584 | 0.649 |
| **SGB14114** | 0.180 | 0.452 | **SGB59869** | 0.586 | 0.628 |
| **SGB47656** | 0.180 | 0.189 | **SGB4727** | 0.587 | 0.700 |
| **SGB6749** | 0.182 | 0.192 | **SGB25431** | 0.591 | 0.664 |
| **SGB14253** | 0.183 | 0.320 | **SGB2299** | 0.592 | 0.320 |
| **SGB15346** | 0.184 | 0.244 | **SGB1829** | 0.593 | 0.495 |
| **SGB4810** | 0.189 | 0.129 | **SGB4990** | 0.597 | 0.600 |
| **SGB4770** | 0.190 | 0.320 | **SGB1891_group** | 0.597 | 0.475 |
| **SGB14043** | 0.191 | 0.401 | **SGB2286** | 0.600 | 0.333 |
| **SGB4886** | 0.192 | 0.464 | **SGB17278** | 0.601 | 0.141 |
| **SGB25497** | 0.192 | 0.224 | **SGB1949** | 0.602 | 0.867 |
| **SGB4815_group** | 0.194 | 0.372 | **SGB63343** | 0.602 | 0.697 |
| **SGB25416** | 0.194 | 0.657 | **SGB5045** | 0.603 | 0.536 |
| **SGB4957** | 0.195 | 0.204 | **SGB5075_group** | 0.603 | 0.571 |
| **SGB4654** | 0.196 | 0.144 | **SGB8007_group** | 0.604 | 0.641 |
| **SGB15373** | 0.199 | 0.257 | **SGB29375** | 0.605 | 0.315 |
| **SGB15254** | 0.205 | 0.118 | **SGB6847** | 0.606 | 0.667 |
| **SGB6571** | 0.205 | 0.293 | **SGB1798** | 0.607 | 0.401 |
| **SGB15323** | 0.207 | 0.355 | **SGB4670** | 0.609 | 0.218 |
| **SGB71759** | 0.207 | 0.164 | **SGB4582_group** | 0.610 | 0.299 |
| **SGB4648** | 0.207 | 0.388 | **SGB4290** | 0.612 | 0.527 |
| **SGB15180** | 0.209 | 0.165 | **SGB14891** | 0.612 | 0.596 |
| **SGB15413** | 0.209 | 0.343 | **SGB1785** | 0.612 | 0.329 |
| **SGB49168** | 0.212 | 0.372 | **SGB15209** | 0.614 | 0.693 |
| **SGB14960** | 0.215 | 0.346 | **SGB14150** | 0.620 | 0.718 |
| **SGB4133** | 0.215 | 0.451 | **SGB33551** | 0.620 | 0.227 |
| **SGB15051** | 0.218 | 0.229 | **SGB14958** | 0.621 | 0.653 |
| **SGB4993** | 0.218 | 0.513 | **SGB14807** | 0.622 | 0.568 |
| **SGB15395** | 0.220 | 0.566 | **SGB4820** | 0.624 | 0.556 |
| **SGB15145** | 0.223 | 0.257 | **SGB1812** | 0.624 | 0.460 |
| **SGB5111** | 0.223 | 0.147 | **SGB4716** | 0.627 | 0.281 |
| **SGB6317** | 0.224 | 0.467 | **SGB4425_group** | 0.627 | 0.461 |
| **SGB4966** | 0.224 | 0.224 | **SGB9340** | 0.628 | 0.377 |
| **SGB4780** | 0.226 | 0.200 | **SGB14779** | 0.629 | 0.716 |
| **SGB14198** | 0.227 | 0.238 | **SGB14125** | 0.630 | 0.634 |
| **SGB63101** | 0.228 | 0.501 | **SGB14259** | 0.631 | 0.567 |
| **SGB4779** | 0.230 | 0.471 | **SGB4784** | 0.631 | 0.862 |
| **SGB15233** | 0.233 | 0.325 | **SGB15260** | 0.633 | 0.369 |
| **SGB4769** | 0.234 | 0.250 | **SGB14741** | 0.634 | 0.821 |
| **SGB2295** | 0.238 | 0.383 | **SGB4577_group** | 0.635 | 0.640 |
| **SGB72336** | 0.240 | 0.609 | **SGB71281** | 0.637 | 0.484 |
| **SGB4658** | 0.241 | 0.170 | **SGB14307** | 0.638 | 0.640 |
| **SGB14770** | 0.245 | 0.446 | **SGB5803** | 0.638 | 0.460 |
| **SGB6148** | 0.247 | 0.603 | **SGB58519** | 0.641 | 0.607 |
| **SGB25493** | 0.247 | 0.301 | **SGB5077** | 0.643 | 0.523 |
| **SGB4831_group** | 0.248 | 0.424 | **SGB15125** | 0.645 | 0.851 |
| **SGB14965** | 0.249 | 0.584 | **SGB15143** | 0.647 | 0.683 |
| **SGB15224** | 0.250 | 0.332 | **SGB4116** | 0.649 | 0.912 |
| **SGB4938** | 0.251 | 0.410 | **SGB4677** | 0.652 | 0.804 |
| **SGB15402** | 0.252 | 0.633 | **SGB15467_group** | 0.654 | 0.523 |
| **SGB15291** | 0.253 | 0.087 | **SGB7202** | 0.655 | 0.351 |
| **SGB9333** | 0.254 | 0.209 | **SGB6178** | 0.656 | 0.686 |
| **SGB4664** | 0.254 | 0.066 | **SGB6796_group** | 0.656 | 0.586 |
| **SGB4906** | 0.254 | 0.216 | **SGB6783_group** | 0.658 | 0.600 |
| **SGB4711** | 0.254 | 0.202 | **SGB1860** | 0.658 | 0.617 |
| **SGB15065** | 0.256 | 0.177 | **SGB4059** | 0.659 | 0.529 |
| **SGB714_group** | 0.256 | 0.466 | **SGB63326** | 0.659 | 0.875 |
| **SGB4772** | 0.257 | 0.285 | **SGB9272_group** | 0.659 | 0.638 |
| **SGB3958** | 0.257 | 0.202 | **SGB15350** | 0.659 | 0.517 |
| **SGB4629** | 0.258 | 0.212 | **SGB14334** | 0.660 | 0.599 |
| **SGB14048** | 0.261 | 0.365 | **SGB1941** | 0.665 | 0.461 |
| **SGB15052** | 0.263 | 0.369 | **SGB16986** | 0.667 | 0.667 |
| **SGB14861** | 0.265 | 0.722 | **SGB14909** | 0.668 | 0.768 |
| **SGB9205** | 0.265 | 0.446 | **SGB1963** | 0.669 | 0.507 |
| **SGB4280** | 0.265 | 0.424 | **SGB4774** | 0.671 | 0.617 |
| **SGB4829** | 0.265 | 0.244 | **SGB14143** | 0.671 | 0.810 |
| **SGB4816** | 0.266 | 0.062 | **SGB15272** | 0.671 | 0.729 |
| **SGB2317** | 0.266 | 0.612 | **SGB29380** | 0.672 | 0.898 |
| **SGB15411** | 0.266 | 0.239 | **SGB4811_group** | 0.675 | 0.695 |
| **SGB5117** | 0.267 | 0.315 | **SGB4595** | 0.676 | 0.524 |
| **SGB14250** | 0.268 | 0.230 | **SGB4834** | 0.676 | 0.184 |
| **SGB14924** | 0.269 | 0.338 | **SGB4030** | 0.679 | 0.677 |
| **SGB4767** | 0.270 | 0.171 | **SGB8071** | 0.679 | 0.772 |
| **SGB6376** | 0.271 | 0.226 | **SGB2311** | 0.680 | 0.470 |
| **SGB4714** | 0.271 | 0.128 | **SGB3991** | 0.680 | 0.663 |
| **SGB4691** | 0.274 | 0.292 | **SGB4722** | 0.681 | 0.685 |
| **SGB14341** | 0.275 | 0.433 | **SGB17244** | 0.682 | 0.536 |
| **SGB15244** | 0.275 | 0.156 | **SGB3993** | 0.682 | 0.648 |
| **SGB5082_group** | 0.276 | 0.115 | **SGB4553** | 0.683 | 0.137 |
| **SGB4910** | 0.277 | 0.431 | **SGB6956** | 0.683 | 0.544 |
| **SGB4914** | 0.277 | 0.232 | **SGB1699** | 0.689 | 0.497 |
| **SGB8599** | 0.279 | 0.275 | **SGB6962_group** | 0.691 | 0.796 |
| **SGB4936** | 0.282 | 0.138 | **SGB4705** | 0.691 | 0.569 |
| **SGB15374** | 0.284 | 0.290 | **SGB1957** | 0.693 | 0.295 |
| **SGB72916** | 0.284 | 0.298 | **SGB4532** | 0.694 | 0.410 |
| **SGB4909** | 0.286 | 0.180 | **SGB4327_group** | 0.695 | 0.450 |
| **SGB15390** | 0.287 | 0.457 | **SGB59559** | 0.698 | 0.803 |
| **SGB15164** | 0.287 | 0.642 | **SGB4594** | 0.699 | 0.683 |
| **SGB15093** | 0.289 | 0.359 | **SGB5765_group** | 0.700 | 0.518 |
| **SGB13983** | 0.291 | 0.452 | **SGB17169** | 0.702 | 0.672 |
| **SGB5042** | 0.292 | 0.425 | **SGB15120** | 0.705 | 0.710 |
| **SGB4771** | 0.293 | 0.194 | **SGB1948** | 0.705 | 0.421 |
| **SGB15356** | 0.295 | 0.114 | **SGB14853** | 0.706 | 0.928 |
| **SGB72479** | 0.296 | 0.247 | **SGB14898** | 0.706 | 0.756 |
| **SGB4557** | 0.296 | 0.218 | **SGB48424** | 0.707 | 0.720 |
| **SGB3988** | 0.297 | 0.525 | **SGB5792** | 0.707 | 0.619 |
| **SGB15041** | 0.298 | 0.265 | **SGB6754** | 0.707 | 0.241 |
| **SGB14128** | 0.298 | 0.427 | **SGB1934** | 0.708 | 0.730 |
| **SGB15385** | 0.299 | 0.296 | **SGB14854** | 0.709 | 0.743 |
| **SGB6750** | 0.300 | 0.287 | **SGB6768** | 0.709 | 0.858 |
| **SGB4184** | 0.302 | 0.331 | **SGB15156_group** | 0.710 | 0.710 |
| **SGB3573** | 0.303 | 0.562 | **SGB4750** | 0.711 | 0.599 |
| **SGB66170** | 0.304 | 0.680 | **SGB14142** | 0.711 | 0.877 |
| **SGB15201** | 0.305 | 0.653 | **SGB17153_group** | 0.712 | 0.787 |
| **SGB15203** | 0.306 | 0.239 | **SGB4552_group** | 0.712 | 0.329 |
| **SGB79798** | 0.307 | 0.574 | **SGB4951** | 0.716 | 0.541 |
| **SGB15382** | 0.309 | 0.533 | **SGB4303** | 0.716 | 0.355 |
| **SGB4652** | 0.310 | 0.300 | **SGB9286** | 0.720 | 0.504 |
| **SGB9346** | 0.311 | 0.343 | **SGB5051** | 0.725 | 0.491 |
| **SGB14969** | 0.311 | 0.191 | **SGB17237** | 0.726 | 0.571 |
| **SGB4262** | 0.313 | 0.440 | **SGB4940** | 0.726 | 0.624 |
| **SGB4394** | 0.313 | 0.248 | **SGB4597** | 0.727 | 0.668 |
| **SGB61601** | 0.314 | 0.529 | **SGB4563_group** | 0.731 | 0.793 |
| **SGB15216** | 0.317 | 0.468 | **SGB1867** | 0.732 | 0.602 |
| **SGB14027** | 0.318 | 0.486 | **SGB47515** | 0.733 | 0.863 |
| **SGB4674** | 0.319 | 0.708 | **SGB59562** | 0.733 | 0.700 |
| **SGB14937** | 0.320 | 0.504 | **SGB8059_group** | 0.739 | 0.631 |
| **SGB15090** | 0.321 | 0.262 | **SGB4991** | 0.740 | 0.780 |
| **SGB9391** | 0.323 | 0.219 | **SGB4540_group** | 0.741 | 0.428 |
| **SGB15383** | 0.324 | 0.231 | **SGB14862** | 0.743 | 0.683 |
| **SGB29347** | 0.324 | 0.693 | **SGB4422** | 0.744 | 0.560 |
| **SGB14991** | 0.324 | 0.311 | **SGB15124** | 0.745 | 0.831 |
| **SGB14940** | 0.324 | 0.360 | **SGB14987** | 0.747 | 0.519 |
| **SGB4809** | 0.326 | 0.440 | **SGB7263** | 0.749 | 0.839 |
| **SGB6141** | 0.327 | 0.384 | **SGB9283** | 0.751 | 0.600 |
| **SGB4687** | 0.332 | 0.370 | **SGB4348** | 0.757 | 0.520 |
| **SGB63163** | 0.333 | 0.607 | **SGB14895** | 0.758 | 0.910 |
| **SGB14177** | 0.334 | 0.407 | **SGB17154** | 0.759 | 0.768 |
| **SGB4832** | 0.334 | 0.244 | **SGB17167** | 0.759 | 0.664 |
| **SGB15160** | 0.335 | 0.234 | **SGB4626** | 0.759 | 0.641 |
| **SGB48024** | 0.335 | 0.237 | **SGB5843** | 0.762 | 0.529 |
| **SGB6179** | 0.336 | 0.460 | **SGB4575** | 0.763 | 0.499 |
| **SGB4768** | 0.342 | 0.308 | **SGB4613** | 0.765 | 0.793 |
| **SGB5090_group** | 0.343 | 0.177 | **SGB15076** | 0.771 | 0.734 |
| **SGB29302** | 0.345 | 0.701 | **SGB17130** | 0.772 | 0.620 |
| **SGB9712_group** | 0.345 | 0.353 | **SGB15904** | 0.774 | 0.585 |
| **SGB3813** | 0.346 | 0.423 | **SGB4080** | 0.775 | 0.814 |
| **SGB79833** | 0.346 | 0.273 | **SGB6846** | 0.777 | 0.499 |
| **SGB4659** | 0.350 | 0.256 | **SGB5825_group** | 0.778 | 0.675 |
| **SGB4328** | 0.350 | 0.283 | **SGB14808** | 0.779 | 0.618 |
| **SGB4776** | 0.350 | 0.722 | **SGB4874** | 0.779 | 0.502 |
| **SGB1790** | 0.351 | 0.783 | **SGB9228** | 0.782 | 0.625 |
| **SGB14313** | 0.351 | 0.315 | **SGB6320** | 0.783 | 0.711 |
| **SGB5043** | 0.351 | 0.364 | **SGB9260** | 0.784 | 0.656 |
| **SGB15127** | 0.352 | 0.626 | **SGB8002** | 0.785 | 0.546 |
| **SGB15049** | 0.352 | 0.229 | **SGB6936** | 0.787 | 0.522 |
| **SGB42321** | 0.353 | 0.453 | **SGB14962** | 0.787 | 0.905 |
| **SGB15403** | 0.353 | 0.592 | **SGB8053** | 0.788 | 0.897 |
| **SGB15115** | 0.363 | 0.191 | **SGB4701** | 0.788 | 0.785 |
| **SGB4905** | 0.363 | 0.186 | **SGB5197** | 0.789 | 0.484 |
| **SGB14838** | 0.363 | 0.878 | **SGB4036** | 0.790 | 0.860 |
| **SGB15012** | 0.365 | 0.189 | **SGB4744** | 0.793 | 0.805 |
| **SGB9202** | 0.368 | 0.350 | **SGB1877** | 0.794 | 0.751 |
| **SGB80143** | 0.369 | 0.144 | **SGB29313** | 0.799 | 0.698 |
| **SGB3992** | 0.370 | 0.757 | **SGB14845** | 0.799 | 0.701 |
| **SGB7259** | 0.372 | 0.625 | **SGB14963** | 0.799 | 0.856 |
| **SGB4546** | 0.373 | 0.341 | **SGB8056** | 0.801 | 0.745 |
| **SGB14974** | 0.373 | 0.188 | **SGB6939** | 0.803 | 0.644 |
| **SGB13976** | 0.374 | 0.613 | **SGB17256** | 0.804 | 0.710 |
| **SGB15342** | 0.376 | 0.529 | **SGB4749** | 0.804 | 0.428 |
| **SGB2296** | 0.377 | 0.484 | **SGB3961** | 0.805 | 0.683 |
| **SGB14941** | 0.377 | 0.493 | **SGB7142** | 0.805 | 0.541 |
| **SGB3996** | 0.379 | 0.508 | **SGB14999** | 0.805 | 0.549 |
| **SGB53497** | 0.380 | 0.739 | **SGB4747** | 0.806 | 0.813 |
| **SGB15470** | 0.381 | 0.727 | **SGB15149** | 0.807 | 0.641 |
| **SGB14020** | 0.381 | 0.427 | **SGB4987** | 0.807 | 0.724 |
| **SGB1858** | 0.382 | 0.342 | **SGB6767** | 0.810 | 0.776 |
| **SGB14851** | 0.386 | 0.919 | **SGB17248** | 0.810 | 0.657 |
| **SGB6305** | 0.388 | 0.439 | **SGB4725** | 0.810 | 0.686 |
| **SGB14932** | 0.388 | 0.636 | **SGB59576** | 0.810 | 0.643 |
| **SGB15089** | 0.389 | 0.577 | **SGB1903_group** | 0.811 | 0.301 |
| **SGB1862** | 0.390 | 0.417 | **SGB5183** | 0.811 | 0.635 |
| **SGB15401** | 0.392 | 0.680 | **SGB49059** | 0.815 | 0.742 |
| **SGB4027** | 0.392 | 0.246 | **SGB4121** | 0.819 | 0.836 |
| **SGB15140** | 0.392 | 0.455 | **SGB25538** | 0.819 | 0.707 |
| **SGB2325** | 0.393 | 0.433 | **SGB3970** | 0.820 | 0.897 |
| **SGB14317** | 0.393 | 0.347 | **SGB3969** | 0.822 | 0.692 |
| **SGB4628** | 0.394 | 0.626 | **SGB14890** | 0.822 | 0.767 |
| **SGB4669** | 0.395 | 0.216 | **SGB1830_group** | 0.824 | 0.578 |
| **SGB15299** | 0.395 | 0.442 | **SGB7967** | 0.824 | 0.341 |
| **SGB6478** | 0.395 | 0.393 | **SGB17168** | 0.825 | 0.650 |
| **SGB14262** | 0.397 | 0.521 | **SGB8047** | 0.828 | 0.839 |
| **SGB63342** | 0.399 | 0.536 | **SGB4046** | 0.829 | 0.720 |
| **SGB4960** | 0.400 | 0.178 | **SGB1855_group** | 0.830 | 0.711 |
| **SGB63333** | 0.400 | 0.365 | **SGB3922** | 0.830 | 0.937 |
| **SGB15316_group** | 0.401 | 0.213 | **SGB14995** | 0.833 | 0.849 |
| **SGB4651** | 0.404 | 0.284 | **SGB7253** | 0.838 | 0.841 |
| **SGB1965** | 0.404 | 0.452 | **SGB48013** | 0.838 | 0.795 |
| **SGB15081** | 0.406 | 0.238 | **SGB4741** | 0.841 | 0.696 |
| **SGB59819** | 0.408 | 0.608 | **SGB4671** | 0.842 | 0.730 |
| **SGB2326** | 0.410 | 0.391 | **SGB15878** | 0.844 | 0.901 |
| **SGB14912** | 0.413 | 0.444 | **SGB6769** | 0.844 | 0.937 |
| **SGB14322_group** | 0.416 | 0.244 | **SGB14180** | 0.844 | 0.770 |
| **SGB3940** | 0.416 | 0.319 | **SGB29433** | 0.846 | 0.728 |
| **SGB4029** | 0.417 | 0.686 | **SGB1871** | 0.846 | 0.624 |
| **SGB2301** | 0.419 | 0.684 | **SGB5182** | 0.852 | 0.594 |
| **SGB63167** | 0.421 | 0.556 | **SGB5736** | 0.853 | 0.875 |
| **SGB14797_group** | 0.422 | 0.353 | **SGB6771** | 0.854 | 0.681 |
| **SGB5200** | 0.428 | 0.577 | **SGB4721** | 0.857 | 0.841 |
| **SGB17347** | 0.431 | 0.377 | **SGB14837** | 0.858 | 0.865 |
| **SGB4868** | 0.432 | 0.667 | **SGB4044** | 0.861 | 0.685 |
| **SGB15067** | 0.433 | 0.452 | **SGB8095** | 0.862 | 0.769 |
| **SGB53515** | 0.434 | 0.469 | **SGB17152** | 0.863 | 0.592 |
| **SGB15075** | 0.435 | 0.359 | **SGB1861** | 0.864 | 0.486 |
| **SGB4421** | 0.436 | 0.380 | **SGB66069** | 0.865 | 0.898 |
| **SGB5121** | 0.437 | 0.131 | **SGB4031** | 0.869 | 0.642 |
| **SGB9226** | 0.438 | 0.372 | **SGB6153** | 0.871 | 0.710 |
| **SGB2318** | 0.439 | 0.773 | **SGB7264** | 0.880 | 0.464 |
| **SGB14894** | 0.441 | 0.815 | **SGB15121** | 0.883 | 0.747 |
| **SGB4817** | 0.443 | 0.257 | **SGB25437** | 0.884 | 0.871 |
| **SGB14966** | 0.443 | 0.278 | **SGB14546_group** | 0.885 | 0.843 |
| **SGB3989** | 0.444 | 0.310 | **SGB4933_group** | 0.888 | 0.635 |
| **SGB15370** | 0.444 | 0.201 | **SGB4763** | 0.888 | 0.906 |
| **SGB14975** | 0.444 | 0.559 | **SGB5193** | 0.889 | 0.961 |
| **SGB4436** | 0.446 | 0.416 | **SGB7985** | 0.891 | 0.674 |
| **SGB14839** | 0.446 | 0.561 | **SGB4699** | 0.892 | 0.843 |
| **SGB14993_group** | 0.451 | 0.127 | **SGB19850_group** | 0.893 | 0.569 |
| **SGB15322** | 0.452 | 0.403 | **SGB17137** | 0.895 | 0.811 |
| **SGB9387** | 0.454 | 0.454 | **SGB4785** | 0.897 | 0.835 |
| **SGB3959** | 0.454 | 0.426 | **SGB15078** | 0.898 | 0.904 |
| **SGB6362** | 0.455 | 0.459 | **SGB53821** | 0.898 | 0.828 |
| **SGB4063** | 0.455 | 0.626 | **SGB15452** | 0.899 | 0.839 |
| **SGB14773_group** | 0.455 | 0.273 | **SGB15271** | 0.900 | 0.893 |
| **SGB29334** | 0.456 | 0.398 | **SGB4724** | 0.902 | 0.818 |
| **SGB14151** | 0.457 | 0.586 | **SGB8255_group** | 0.906 | 0.857 |
| **SGB15087** | 0.457 | 0.419 | **SGB79823** | 0.906 | 0.647 |
| **SGB14022** | 0.458 | 0.526 | **SGB8028_group** | 0.908 | 0.804 |
| **SGB14972** | 0.459 | 0.166 | **SGB4573_group** | 0.912 | 0.768 |
| **SGB15045** | 0.459 | 0.400 | **SGB14874** | 0.913 | 0.964 |
| **SGB4712** | 0.460 | 0.209 | **SGB4988** | 0.914 | 0.679 |
| **SGB15389** | 0.463 | 0.518 | **SGB5184** | 0.914 | 0.894 |
| **SGB82503** | 0.468 | 0.741 | **SGB4786** | 0.915 | 0.713 |
| **SGB15318_group** | 0.469 | 0.479 | **SGB4826_group** | 0.915 | 0.840 |
| **SGB1857** | 0.470 | 0.412 | **SGB4041** | 0.916 | 0.918 |
| **SGB4828_group** | 0.470 | 0.333 | **SGB7984** | 0.917 | 0.584 |
| **SGB4788_group** | 0.470 | 0.170 | **SGB4761** | 0.919 | 0.777 |
| **SGB79822** | 0.471 | 0.344 | **SGB4447** | 0.922 | 0.524 |
| **SGB4269** | 0.473 | 0.210 | **SGB6744** | 0.923 | 0.757 |
| **SGB14923** | 0.473 | 0.622 | **SGB1836_group** | 0.926 | 0.546 |
| **SGB2328** | 0.474 | 0.625 | **SGB1814** | 0.926 | 0.778 |
| **SGB1784** | 0.474 | 0.350 | **SGB4630_group** | 0.927 | 0.885 |
| **SGB14137** | 0.475 | 0.686 | **SGB8163** | 0.930 | 0.778 |
| **SGB15204** | 0.478 | 0.489 | **SGB4617** | 0.933 | 0.866 |
| **SGB7256** | 0.485 | 0.698 | **SGB4588_group** | 0.933 | 0.784 |
| **SGB15295_group** | 0.485 | 0.191 | **SGB4742** | 0.937 | 0.660 |
| **SGB14182** | 0.486 | 0.341 | **SGB4572** | 0.938 | 0.900 |
| **SGB29342** | 0.488 | 0.763 | **SGB10115** | 0.938 | 0.731 |
| **SGB4438** | 0.488 | 0.614 | **SGB15158** | 0.942 | 0.878 |
| **SGB14824_group** | 0.489 | 0.260 | **SGB29328** | 0.943 | 0.840 |
| **SGB2303** | 0.489 | 0.620 | **SGB4791** | 0.945 | 0.942 |
| **SGB9262** | 0.489 | 0.309 | **SGB4688** | 0.946 | 0.901 |
| **SGB14952** | 0.490 | 0.700 | **SGB10068** | 0.949 | 0.825 |
| **SGB14951** | 0.491 | 0.542 | **SGB71883** | 0.957 | 0.788 |
| **SGB15459** | 0.493 | 0.591 | **SGB4760** | 0.959 | 0.818 |
| **SGB6358** | 0.494 | 0.271 | **SGB4037_group** | 0.961 | 0.909 |
| **SGB14929** | 0.494 | 0.267 | **SGB4529** | 0.964 | 0.922 |
| **SGB15286** | 0.495 | 0.604 | **SGB4837_group** | 0.968 | 0.781 |
| **SGB5060** | 0.495 | 0.345 | **SGB79883** | 0.968 | 0.969 |
| **SGB15332_group** | 0.497 | 0.356 | **SGB4762** | 0.968 | 0.796 |
| **SGB15126** | 0.497 | 0.554 | **SGB4797** | 0.974 | 0.913 |
| **SGB72433_group** | 0.498 | 0.297 | **SGB14809** | 0.977 | 0.872 |
| **SGB4045** | 0.498 | 0.498 | **SGB4758_group** | 0.978 | 0.891 |
| **SGB1626** | 0.499 | 0.579 | **SGB4703** | 0.978 | 0.959 |
| **SGB5180** | 0.499 | 0.422 | **SGB4606** | 0.981 | 0.874 |
| **SGB4867** | 0.499 | 0.426 | **SGB4584** | 0.981 | 0.932 |
| **SGB4825** | 0.506 | 0.108 | **SGB15132** | 0.981 | 0.901 |
| **SGB4925** | 0.506 | 0.397 | **SGB4746** | 0.985 | 0.809 |
| **SGB53517** | 0.507 | 0.679 | **SGB4862** | 0.986 | 0.893 |
| **SGB1844** | 0.508 | 0.335 | **SGB4798** | 0.986 | 0.755 |
| **SGB14844** | 0.515 | 0.747 | **SGB4861** | 0.989 | 0.943 |
| **SGB4871_group** | 0.516 | 0.289 | **SGB4608** | 0.991 | 0.904 |
| **SGB14050** | 0.518 | 0.460 | **SGB4035** | 0.994 | 0.968 |
| **SGB25547** | 0.519 | 0.683 | **SGB4794_group** | 0.996 | 0.956 |
| **SGB1815** | 0.519 | 0.292 | **SGB4753** | 0.997 | 0.928 |
| **SGB3957** | 0.519 | 0.631 | **SGB4583** | 1.000 | 0.965 |
| **SGB54347** | 0.519 | 0.729 | | | |

**Table 1B - identification and ranking of select pro-health indicator microbes**

| **SGB** | **Health Rank** | **Health and Diet Rank** | **Known / Unknown** | **Level taxonomy** | **Species Label** |
|---|---|---|---|---|---|
| SGB15249 | 0.01295 | 0.03788 | kSGB | Species | s_Ruminococcaceae_bacterium |
| SGB6340 | 0.01448 | 0.01144 | uSGB | Family | s_GGB4585_SGB6340 |
| SGB4964 | 0.03551 | 0.01271 | kSGB | Species | s_Lachnospiraceae_bacterium |
| SGB14252 | 0.04653 | 0.03567 | kSGB | Species | s_Clostridia_bacterium |
| SGB15229 | 0.05230 | 0.04529 | uSGB | Family | s_GGB9707_SGB15229 |
| SGB6174 group | 0.05683 | 0.05821 | kSGB | Species | s_Clostridium_sp_NSJ_42 |
| SGB15317 | 0.05939 | 0.02016 | kSGB | Species | s_Faecalibacterium_prausni tzii |
| SGB14179 | 0.07117 | 0.11379 | uSGB | Other | s_GGB9237_SGB14179 |
| SGB15225 | 0.07187 | 0.07097 | uSGB | Family | s_GGB9705_SGB15225 |
| SGB4894 | 0.07226 | 0.06731 | uSGB | Genus | s_Lachnospiraceae_unclassified SGB4894 |
| SGB4643 | 0.07249 | 0.04395 | uSGB | Family | s_GGB3478_SGB4643 |
| SGB4963 | 0.07346 | 0.05215 | kSGB | Species | s_Lachnospiraceae_bacterium |
| SGB79840 | 0.07347 | 0.06468 | kSGB | Species | s_Intestinimonas_gabonensis |
| SGB4893 | 0.07503 | 0.04669 | kSGB | Species | s_Lachnospiraceae_bacterium OM04_12BH |
| SGB6276 | 0.07948 | 0.09152 | uSGB | Genus | s_Clostridia_unclassified_SGB6276 |
| SGB3952 | 0.08883 | 0.16062 | kSGB | Species | s_Clostridia_bacterium |
| SGB4638 | 0.08937 | 0.12535 | kSGB | Species | s_Lachnospiraceae_bacterium |
| SGB15236 | 0.09254 | 0.11862 | uSGB | Genus | s_Ruminococcaceae_unclassified SGB15236 |
| SGB4191 | 0.09427 | 0.12455 | uSGB | Genus | s_Ruminococcaceae_unclassified SGB4191 |
| SGB15053 group | 0.09860 | 0.07499 | uSGB | Family | s_GGB9615_SGB15053 |
| SGB15368 | 0.10061 | 0.06770 | uSGB | Family | s_GGB9758_SGB15368 |
| SGB4782 | 0.10281 | 0.04146 | kSGB | Species | s_Lachnospiraceae_bacterium |
| SGB14042 | 0.10312 | 0.11185 | kSGB | Species | s_Clostridia_bacterium |
| SGB4706 | 0.10943 | 0.05344 | kSGB | Species | s_Lachnospiraceae_bacterium |
| SGB4644 | 0.11568 | 0.10116 | kSGB | Species | s_Clostridium_sp_AF36_4 |
| SGB49188 | 0.11574 | 0.23073 | kSGB | Species | s_Firmicutes_bacterium |
| SGB4781 | 0.11872 | 0.11708 | kSGB | Species | s_Lachnospiraceae_bacterium |
| SGB4777 | 0.11931 | 0.05191 | uSGB | Family | s_GGB3570_SGB4777 |
| SGB14921 | 0.12060 | 0.16745 | uSGB | Family | s_GGB9522_SGB14921 |
| SGB15234 | 0.12447 | 0.17389 | uSGB | Genus | s_Ruminococcaceae_unclassified SGB15234 |
| SGB8601 | 0.12830 | 0.13841 | kSGB | Species | s_Candidatus_Gastranaerophilales bacterium |
| SGB5087 | 0.12934 | 0.07516 | kSGB | Species | s_Lachnospira_sp_NSJ_43 |
| SGB14311 | 0.13091 | 0.21316 | kSGB | Species | s_Clostridia_bacterium |
| SGB4953 | 0.13203 | 0.06709 | kSGB | Species | s_Lachnospiraceae_bacterium |
| SGB7258 | 0.13209 | 0.11251 | kSGB | Species | s_Oscillibacter_sp_PC13 |
| SGB4882 | 0.13224 | 0.09517 | uSGB | Genus | s _Lachnospiraceae_unclassified SGB4882 |
| SGB6367 | 0.13257 | 0.08855 | uSGB | Family | s_GGB4603_SGB6367 |
| SGB15106 | 0.13443 | 0.14450 | uSGB | Family | s_GGB9635_SGB15106 |
| SGB4778 | 0.14045 | 0.08654 | uSGB | Family | s_GGB3571_SGB477 |
| SGB15131 | 0.14152 | 0.25835 | kSGB | Species | s_Lawsonibacter_sp_NSJ_51 |
| SGB4198 group | 0.14354 | 0.25145 | kSGB | Species | s_Eubacterium_siraeum |
| SGB15031 | 0.15023 | 0.19720 | uSGB | Family | s_GGB9602_SGB 15031 |
| SGB13981 | 0.15359 | 0.15525 | kSGB | Species | s_Clostridia_bacterium |
| SGB15123 | 0.16242 | 0.19465 | uSGB | Family | s_GGB9646_SGB15123 |
| SGB54300 | 0.16384 | 0.17829 | kSGB | Species | s_Clostridia_bacterium |
| SGB4665 | 0.16585 | 0.08980 | uSGB | Other | s_GGB3491_SGB4665 |
| SGB13979 | 0.16638 | 0.11780 | kSGB | Species | s_Clostridia_bacterium |
| SGB15410 | 0.16878 | 0.14887 | uSGB | Family | s_GGB9787_SGB15410 |
| SGB2290 | 0.16936 | 0.20167 | kSGB | Species | s_Alistipes_communis |
| SGB14954 | 0.16999 | 0.23594 | kSGB | Species | s_Clostridia_bacterium |
| SGB14306 | 0.17277 | 0.19560 | uSGB | Family | s_GGB9342_SGB14306 |
| SGB4805 | 0.17317 | 0.28601 | uSGB | Genus | s_Blautia_SGB4805 |
| SGB14899 | 0.17381 | 0.37839 | kSGB | Species | s_Ruminococcaceae_bacterium |
| SGB4803 | 0.17407 | 0.20513 | kSGB | Species | s_Lachnospiraceae_bacterium |
| SGB13982 | 0.17658 | 0.21446 | kSGB | Species | s_Clostridia_bacterium |
| SGB15265 group | 0.17739 | 0.13848 | uSGB | Genus | s_Ruminococcaceae_unclassified SGB 15265 |
| SGB14114 | 0.17952 | 0.29401 | uSGB | Family | s_GGB9176_SGB14114 |
| SGB47656 | 0.17999 | 0.10019 | kSGB | Species | s_Lachnospiraceae_bacterium_NSJ_46 |
| SGB6749 | 0.18194 | 0.11580 | kSGB | Species | s_Clostridium_saccharogumia |
| SGB14253 | 0.18296 | 0.14263 | kSGB | Species | s_Clostridia_bacterium |
| SGB15346 | 0.18435 | 0.11324 | uSGB | Genus | s_Faecalibacterium_SGB15346 |
| SGB4810 | 0.18853 | 0.08351 | kSGB | Species | s_Blautia_sp_AF19_10LB |
| SGB4770 | 0.18992 | 0.13870 | kSGB | Species | s_Clostridiaceae_bacterium |
| SGB25497 | 0.19225 | 0.11007 | kSGB | Species | s_Ruminococcus_sp_AF41_9 |
| SGB4957 | 0.19498 | 0.10400 | kSGB | Species | s_Lachnospiraceae_bacterium |
| SGB4654 | 0.19578 | 0.11904 | kSGB | Species | s_Roseburia_sp_AM59_24XD |
| SGB15373 | 0.19896 | 0.14592 | kSGB | Species | s_Clostridia_bacterium |
| SGB15254 | 0.20473 | 0.14108 | kSGB | Species | s_Oscillibacter_sp_ER4 |
| SGB15323 | 0.20657 | 0.14707 | kSGB | Species | s_Faecalibacterium_prausnitzii |
| SGB71759 | 0.20708 | 0.10113 | uSGB | Other | s_GGB51441_SGB71759 |
| SGB15180 | 0.20880 | 0.12347 | uSGB | Family | s_GGB9677_SGB15180 |
| SGB49168 | 0.21242 | 0.13318 | kSGB | Species | s_Peptococcaceae_bacterium |
| SGB15051 | 0.21791 | 0.11890 | uSGB | Family | s_GGB9615_SGB 15051 |
| SGB15145 | 0.22275 | 0.14522 | uSGB | Genus | s_Clostridiales_unclassified_SGB15145 |
| SGB4966 | 0.22442 | 0.12453 | kSGB | Species | s_Lachno spiraceae_bacterium_OF 0 9_6 |
| SGB4780 | 0.22571 | 0.11311 | kSGB | Species | s_Lachnospiraceae_bacterium |
| SGB15291 | 0.25287 | 0.14375 | uSGB | Family | s_GGB9730_SGB15291 |
| SGB4816 | 0.26564 | 0.13167 | kSGB | Species | s_Blautia_glucerasea |
| SGB4714 | 0.27136 | 0.11458 | kSGB | Species | s_Clostridium_sp_AF20_17LB |

**Table 1C: identification and ranking of select poor-health indicator microbes**

| **SGB** | **Health Rank** | **Health and Diet Rank** | **Known / Unknown** | **Level taxonomy** | **Species Label** |
|---|---|---|---|---|---|
| SGB7253 | 0.83757 | 0.90439 | kSGB | Species | s_Massilimaliae_timonensis |
| SGB6769 | 0.84367 | 0.90635 | kSGB | Species | s_Longibaculum_muris |
| SGB4721 | 0.85684 | 0.92161 | kSGB | Species | s_Clostridiaceae_bacterium |
| SGB14837 | 0.85756 | 0.91502 | kSGB | Species | s_Phocea_massiliensis |
| SGB14546 group | 0.88488 | 0.90584 | kSGB | Species | s_Collinsella_aerofaciens |
| SGB4763 | 0.88841 | 0.90816 | kSGB | Species | s_Clostridiales_bacterium_1_7_47FAA |
| SGB5193 | 0.88927 | 0.92589 | kSGB | Species | s_Anaerotignum_lactatifermentans |
| SGB7985 | 0.89132 | 0.90473 | kSGB | Species | s_Lactococcus_lactis |
| SGB4699 | 0.89204 | 0.91691 | kSGB | Species | s_Clostridium_symbiosum |
| SGB19850 group | 0.89309 | 0.86880 | uSGB | Genus | s_Candidatus_Saccharibacteria unclassified_SGB 19850 |
| SGB17137 | 0.89514 | 0.89471 | kSGB | Species | s_Trueperella_pyogenes |
| SGB4785 | 0.89737 | 0.90411 | kSGB | Species | s_Blautia_producta |
| SGB15078 | 0.89839 | 0.94997 | kSGB | Species | s_Dysosmobacter_welbionis |
| SGB53821 | 0.89842 | 0.90151 | kSGB | Species | s_Ruminococcaceae_bacterium |
| SGB15452 | 0.89858 | 0.93212 | kSGB | Species | s_Bilophila_wadsworthia |
| SGB15271 | 0.90009 | 0.94777 | kSGB | Species | s_Ruthenibacterium_lactatiformans |
| SGB4724 | 0.90225 | 0.90878 | kSGB | Species | s_Enterocloster_asparagiformis |
| SGB8255 group | 0.90607 | 0.90838 | kSGB | Species | s_Streptococcus_sp_263_SSPC |
| SGB79823 | 0.90629 | 0.85359 | uSGB | Other | s_GGB9581_SGB79823 |
| SGB8028 group | 0.90753 | 0.90718 | kSGB | Species | s_Streptococcus_anginosus |
| SGB4573 group | 0.91196 | 0.94165 | uSGB | Family | s_GGB3433_SGB4573 |
| SGB14874 | 0.91337 | 0.94413 | kSGB | Species | s_Clostridia_bacterium |
| SGB4988 | 0.91410 | 0.92728 | kSGB | Species | s_Eisenbergiella_tayi |
| SGB5184 | 0.91435 | 0.94303 | kSGB | Species | s_Clostridia_bacterium |
| SGB4786 | 0.91528 | 0.91554 | kSGB | Species | s Blautia_producta |
| SGB4826 group | 0.91549 | 0.91263 | kSGB | Species | s_Blautia_massiliensis |
| SGB4041 | 0.91577 | 0.94821 | kSGB | Species | s_Longicatena_caecimuris |
| SGB7984 | 0.91695 | 0.88474 | kSGB | Species | s_Lactococcus_lactis |
| SGB4761 | 0.91928 | 0.91746 | kSGB | Species | s_Enterocloster_citroniae |
| SGB4447 | 0.92222 | 0.80227 | uSGB | Genus | s_Clostridia_unclassified_SGB4447 |
| SGB6744 | 0.92315 | 0.92052 | kSGB | Species | s_Erysipelatoclostridium_ramosum |
| SGB 1836 group | 0.92607 | 0.88379 | kSGB | Species | s_Bacteroides_uniformis |
| SGB1814 | 0.92627 | 0.91327 | kSGB | Species | s_Phocaeicola_vulgatus |
| SGB4630 group | 0.92731 | 0.94260 | kSGB | Species | s_Clostridium_scindens |
| SGB8163 | 0.93004 | 0.94153 | kSGB | Species | s_Streptococcus_mitis |
| SGB4617 | 0.93264 | 0.94966 | kSGB | Species | s_Sellimonas_intestinalis |
| SGB4588 group | 0.93315 | 0.94320 | kSGB | Species | s_Tyzzerella_nexilis |
| SGB4742 | 0.93729 | 0.92385 | kSGB | Species | s_Hungatella_hathewayi |
| SGB4572 | 0.93762 | 0.94247 | kSGB | Species | s_Dorea_phocaeensis |
| SGB10115 | 0.93780 | 0.87910 | kSGB | Species | s_Klebsiella_pneumoniae |
| SGB15158 | 0.94161 | 0.97077 | kSGB | Species | s_Clostridiales_bacterium |
| SGB29328 | 0.94283 | 0.93256 | kSGB | Species | s_Clostridium_sp_SN20 |
| SGB4791 | 0.94507 | 0.95441 | kSGB | Species | s_Blautia_producta |
| SGB4688 | 0.94623 | 0.95159 | kSGB | Species | s_Lachnospiraceae_bacterium |
| SGB 10068 | 0.94856 | 0.94769 | kSGB | Species | s_Escherichia_coli |
| SGB71883 | 0.95708 | 0.94116 | uSGB | Other | s_GGB51510_SGB71883 |
| SGB4760 | 0.95906 | 0.95790 | kSGB | Species | s_Enterocloster_clostridioformis |
| SGB4037 group | 0.96056 | 0.96955 | kSGB | Species | s_Clostridium_innocuum |
| SGB4529 | 0.96396 | 0.96707 | kSGB | Species | s_Anaerostipes_caccae |
| SGB4837 group | 0.96774 | 0.94102 | kSGB | Species | s_Blautia_wexlerae |
| SGB79883 | 0.96809 | 0.97395 | uSGB | Family | s_GGB58233_SGB79883 |
| SGB4762 | 0.96812 | 0.96850 | kSGB | Species | s_Enterocloster_aldensis |
| SGB4797 | 0.97424 | 0.97337 | kSGB | Species | s_Blautia_argi |
| SGB 14809 | 0.97745 | 0.97986 | kSGB | Species | s_Eggerthella_lenta |
| SGB4758 group | 0.97753 | 0.98572 | kSGB | Species | s_Enterocloster_bolteae |
| SGB4703 | 0.97771 | 0.97923 | uSGB | Family | s_GGB3523_SGB4703 |
| SGB4606 | 0.98061 | 0.98800 | kSGB | Species | s_Mediterraneibacter_glycyrrhizinilyticus |
| SGB4584 | 0.98107 | 0.98931 | kSGB | Species | s_Ruminococcus_gnavus |
| SGB15132 | 0.98122 | 0.98972 | kSGB | Species | s_Flavonifractor_plautii |
| SGB4746 | 0.98485 | 0.98698 | kSGB | Species | s_Lachnospiraceae_bacterium |
| SGB4862 | 0.98588 | 0.99013 | kSGB | Species | s_Blautia_caecimuris |
| SGB4798 | 0.98632 | 0.96497 | kSGB | Species | s_Blautia_hansenii |
| SGB4861 | 0.98943 | 0.98632 | kSGB | Species | s_Lachnospiraceae_bacterium |
| SGB4608 | 0.99077 | 0.99371 | kSGB | Species | s_Ruminococcus_torques |
| SGB4035 | 0.99414 | 0.99342 | kSGB | Species | s_Amedibacillus_dolichus |
| SGB4794 group | 0.99616 | 0.99312 | kSGB | Species | s_Blautia_hansenii |
| SGB4753 | 0.99668 | 0.99709 | kSGB | Species | s_Clostridium_sp_AT4 |
| SGB4583 | 1.00000 | 0.99928 | kSGB | Species | s_Faecalimonas_umbilicata |

The invention also provides a method of reducing the risk of disease in a human using the food composition disclosed herein. The invention provides a food composition disclosed herein for use in a method of reducing the risk of disease in a human.

The invention also provides a method of improving the general well-being of a human using the food composition disclosed herein. The invention provides a food composition disclosed herein for use in a method of improving the general well-being of a human.

The invention also provides a method of controlling or treating obesity in a human using the food composition disclosed herein. The invention provides a food composition disclosed herein for use in a method of controlling or treating obesity in a human.

The invention also provides a method of achieving and maintaining a healthy body weight in a human using the food composition disclosed herein. The invention provides a food composition disclosed herein for use in a method of achieving and maintaining a healthy body weight in a human.

The invention also provides a method of achieving weight loss in a human using the food composition disclosed herein. The invention provides a food composition disclosed herein for use in a method of achieving weight loss in a human.

The invention also provides a method of reducing inflammation in a human using the food composition disclosed herein. The invention provides a food composition disclosed herein for use in a method of reducing inflammation in a human. Inflammation may be assessed by measuring relative levels of circulating metabolites, such as glycoprotein acetyls (GlycA). For example, reduction in inflammation may be determined by measuring a reduction in GlycA.

The invention also provides a method of improving the glycaemic profile of a human using the food composition disclosed herein. The invention provides a food composition disclosed herein for use in a method of improving the glycaemic profile of a human.

The invention also provides a method of improving the lipaemic profile of a human using the food composition disclosed herein. The invention provides a food composition disclosed herein for use in a method of improving the lipaemic profile of a human. The lipaemic profile may be assessed by measuring relative levels of circulating metabolites, such as apolipoproteinB (ApoB) and LDL-cholesterol (LDL-C). For example, an improved lipaemic profile may be determined by measuring a reduction in ApoB and LDL-C.

The invention also provides a method of treating gastrointestinal symptoms, for example, irritable bowel syndrome using the food composition disclosed herein. The invention provides a food composition disclosed herein for use in a method of treating gastrointestinal symptoms, for example, constipation and bloating, in a human.

The invention also provides a method of treating indigestion using the food composition disclosed herein. The invention provides a food composition disclosed herein for use in a method of treating indigestion.

The invention also provides a method of improving the bowel function of a human using the food composition disclosed herein. The invention also provides a method of improving the mood of a human using the food composition disclosed herein. The invention also provides a method of improving the skin quality of a human using the food composition disclosed herein. The invention also provides a method of improving the satiety of a human using the food composition disclosed herein. The invention also provides a method of improving the sleep quality of a human using the food composition disclosed herein. The invention also provides a method of strengthening the immune system of a human using the food composition disclosed herein. The invention provides a food composition disclosed herein for use in any one of the aforementioned methods.

The invention also provides a method of improving energy levels of a human using the food composition disclosed herein. The invention also provides a food composition disclosed herein for use in improving energy levels of a human.

The invention also provides a method of improving the severity of rumbling stomach using the food composition disclosed herein. The invention also provides a method of reducing postprandial hunger, increasing postprandial energy or increasing postprandial satiety, using the food composition disclosed herein.

The methods disclosed herein require that the food composition is ingested (i.e. consumed) by the human. The food composition disclosed herein may be sprinkled on top of food items, including solid and liquid food, and then ingested by the human. The food composition disclosed herein may be incorporated into food items during their manufacture. For example, the food composition disclosed herein may be mixed into the ingredients of another food item. Such a food item would be said to contain the food composition disclosed herein.

The daily dosage of the food composition refers to the amount of food composition that is required to achieve the desired effect of the methods disclosed herein.

The food composition may be ingested at a daily dosage of at least about 10, 11, 12, 13, 14, 15, 16, 17, 18, 19 or 20 grams. Preferably, the food composition is ingested at a daily dosage of about 15 grams.

The daily dosage may comprise at least about 1, 2, 3, 4, 5, 6, 7, 8, 9 or 10 grams of dietary fibre. Preferably, the daily dosage comprises about 5 grams of dietary fibre.

The invention also provides a composition as described herein, for use as a medicament. In certain embodiments, the composition is for use in a method of one of the preceding paragraphs, in particular for use in controlling or treating obesity, reducing inflammation (e.g. reducing GlycA), improving a glycaemic profile, improving a lipaemic profile (e.g. reducing ApoB or LDL-C), treating gastrointestinal symptoms (such as constipation and bloating), improving bowel function, improving mood, improving skin quality, improving satiety, improving sleep quality, or strengthening the immune system of a human.

The invention also provides use of composition as described herein for the manufacture of a medicament for the treatment of a condition discussed above, in particular for controlling or treating obesity, reducing inflammation (e.g. reducing GlycA), improving a glycaemic profile, improving a lipaemic profile (e.g. reducing ApoB or LDL-C), treating gastrointestinal symptoms (such as constipation and bloating), improving bowel function, improving mood, improving skin quality, improving satiety, improving sleep quality, or strengthening the immune system of a human. The methods and compositions of the invention may be used in combination with a food guidance program that is conveyed to a subject and that is prepared at least in part based on or in reference to a database of biomarkers and other information related to, for instance, general health, gut health, microbiome content (such as presence, absence, abundance, or relative abundance of specific microbes, and/or overall diversity, and other recognized measures of microbiome health), nutritional information, circadian rhythm, gathered from a plurality of individuals. Methods to prepare such an aggregated database of information are known, including methods developed by Zoe Limited. Additional guidance regarding representative methods to generate a database useful in preparing food guidance for a group or an individual (e.g., a personalized food guidance) may be found for instance in: WO 2019/155436 "Generating Predicted Values of Biomarkers For Scoring Food"; WO 2019/155437 "Generating Personalized Nutritional Recommendations Using Predicted Values Of Biomarkers"; WO 2019/224308 "Improving the Accuracy of Measuring Nutritional Responses in a Non-Clinical Setting"; WO 2020/043702 "Generating Personalized Food Recommendations from Different Food Sources"; WO 2020/043705 "Improving The Accuracy of Test Data Outside the Clinic"; WO 2020/043706 "Using at Home Measures to Predict Clinical State and Improving the Accuracy of At Home Measurements/Predictions Data Associated with Circadian Rhythm and Meal Timing"; WO 2021/038530 "Generalized Personalized Food Guidance Using Predicted Food Responses"; US 2021-0065873 A1 "Generating Personalized Food Guidance Using Predicted Food Responses"; and WO 2021/186047 A1 "Microbiome Fingerprints, Dietary Fingerprints, and Microbiome Ancestry, and Methods of their Use".

### General Definitions

Unless defined otherwise, technical and scientific terms used herein have the same meaning as commonly understood by a person skilled in the art to which this disclosure belongs.

As used in this specification and the appended claims, the singular forms "a", "an", and "the" include plural referents unless the content clearly dictates otherwise. Thus, for example, reference to "an additive" includes two or more additives, and the like.

In general, the term "comprising" is intended to mean including but not limited to. For example, the phrase a composition "comprising" particular ingredients, should be interpreted to mean that the composition includes those ingredients, but the composition may comprise further ingredients.

In some aspects of the disclosure, the word "comprising" is replaced with the phrase "consisting of". The term "consisting of" is intended to be limiting.

The term "about" or "around" when referring to a value refers to that value but within a reasonable degree of scientific error. Optionally, a value is "about x" or "around x" if it is within 10%, within 5%, or within 1% of x.

The term "between" in relation to a pair of reference numerical values and its grammatical equivalents as used herein can include the numerical values themselves and the range of values between the reference numerical values.

It is to be understood that different applications of the disclosed methods and products may be tailored to the specific needs in the art. It is also to be understood that the terminology used herein is for the purpose of describing particular embodiments of the disclosure only, and is not intended to be limiting.

All publications, patents and patent applications cited herein, whether supra or infra, are hereby incorporated by reference in their entirety.

The following Examples illustrate the invention.

### Example 1

The following exemplary wholefood prebiotic topping composition was prepared.

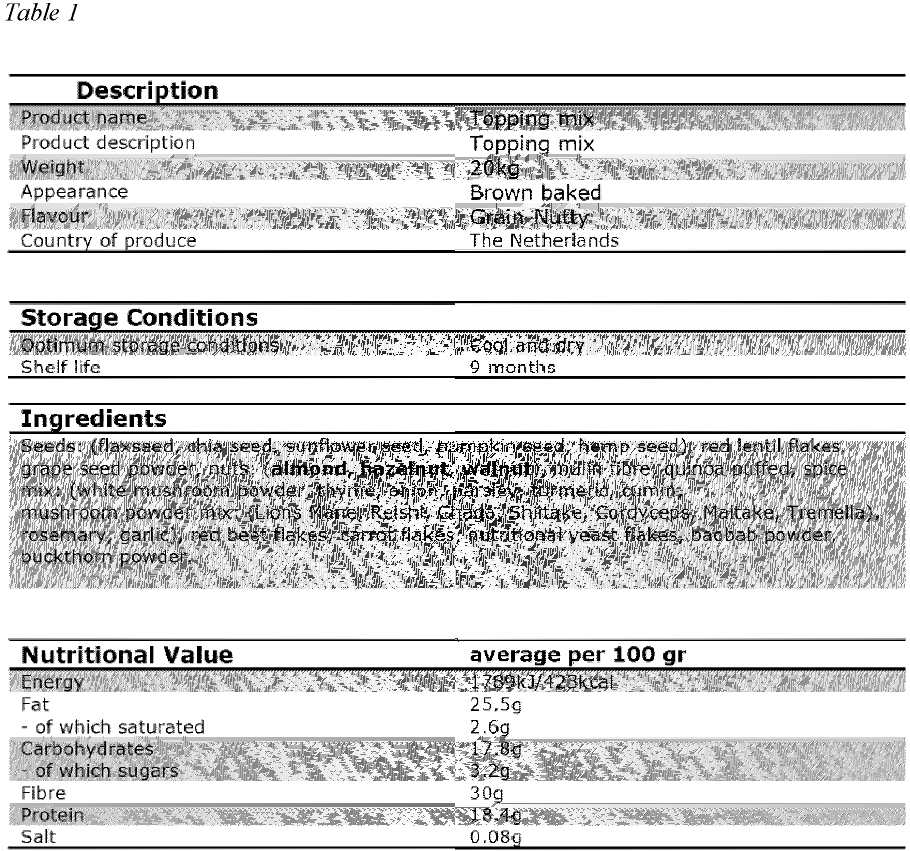

This product is gluten free (contains less than 20ppm gluten).

The nuts and seeds are nibbed, to maintain their structural matrix.

The product is suitable for sprinkling on food items, as part of a normal habitual diet.

The exemplary wholefood prebiotic topping composition comprises the following nutrient profile.

**Table 2**

| Nutrient | `High in' or `source of | Amount in toppers per 100g | % RNI (UK, males) per 100g |
|---|---|---|---|
| Omega 3 ALAs | High in | 1.69g | N/A |
| Iron | High in | 7.9mg | 91% |
| Magnesium | High in | 277mg | 92% |
| Zinc | High in | 5.4mg | 56% |
| Folate | High in | 123ug | 62% |

### Example 2

A randomised controlled trial is carried out, investigating the effects of the prebiotic food composition, in particular on gut health changes, and consumption feasibility.

Host health will be measured through microbiome composition, gastrointestinal symptoms, and subjective hunger, energy and mood. The study design will make use of pre- and probiotic products that contrast in their mechanistic interaction with the gut microbiome and their mode of consumption as a whole food or a dietary supplement. It is hypothesised that the prebiotic intervention arm, containing diverse ingredients and functionally acting as a food that can be added to the participant diet, will offer a wider range of potential benefits, and will show the greatest efficacy in improved host health compared to the other treatments, in particular a probiotic capsule-based intervention containing a single probiotic strain, and a negative control arm (whole-food based salted crouton control).
- Population under investigation - Healthy adults in the UK.
- Data collection spans 7 weeks (including 1 baseline week, and 6 weeks of active treatment consumption in randomised-controlled format).
- Primary objective - To understand the efficacy of the prebiotic food composition on individuals' gut microbiome composition, compared to control treatments. The primary outcome measured is the ZOE microbiome health score. This is derived from the metagenomics analysis of the stool sample, and is calculated by evaluating the presence and proportions of a predefined list of 50 beneficial microbes ("pro-health indicator microbes") and 50 detrimental microbes ("poor health indicator microbes").
- Secondary objectives - Lipaemic, glycaemic and inflammatory profile; gastrointestinal symptoms, bowel habits, mood, hunger, energy, sleep quality, skin quality.
- 360 participants will be randomised in total.
- End point measurements will take place at 6 weeks following the start of treatment.
- Main inclusion criteria - BMI between 18.5kg/m² and 40kg/m², aged between 35 and 65 years old, able to provide written informed consent and comply with the study protocol, have completed the PREDICT Food Frequency Questionnaire sent to them, have not previously completed ZOE.

### - Main exclusion criteria -

o Cannot eat the test treatments safely and comfortably (suffer from inflammatory bowel disease, coeliac disease, Crohn's disease, irritable bowel syndrome, allergies or intolerances, chronic constipation or chronic diarrhoea)
o Have a BMI of less than18.5kg/m² or more than 40kg/m²
o Follow a non-omnivore diet (vegan, vegetarian)
o Have high fermented food intake at baseline for the preceding month (≥7 servings per week)
o Have high fibre intake at baseline for the preceding month (≥20g per day)
o Taking medication or products in the last 3months that may modify the measured study outcomes (Antibiotics, non-topical Steroids or other immunosuppressive medicines, Biologics, Probiotics/Prebiotics, Metformin, Chronic use of non-steroidal anti-inflammatory drugs or opiate pain medicine)
o Have used opiate pain medicine for 8 or more days during the last 3 months
o Have used a proton pump inhibitor for 8 or more days during the last 3 months
o Are currently a smoker
o Have experienced a heart attack, stroke, or major surgery in last 2 months
o Have received treatment for cancer in the last 3 months
o Are currently pregnant, breast feeding or planning a pregnancy
o Are suffering from eating disorders, type 1 or type 2 diabetes mellitus.
   - A statistical analysis will determine the difference between individuals (inter-individual variation) at baseline and endpoint, and between the average changes between treatment arms.

### - Data to be collected & associated storage arrangements -

o Dried bloods pots, stored at room temperature by participants in collection devices and shipped on the same day to a specialized metabolomics lab
   ▪ Metabolomic blood panel - Lipaemic profile, glycaemic profile, inflammatory profile - baseline, endpoint
o Stool samples, stored at room temperature by participants in collection devices and shipped on the same day to the analyzing laboratory
o Non-tissue data will also be collected, through remotely administered online questionnaires (weekly unless noted) -
   ▪ Digestive symptoms and bowel habits - bloating, abdominal pain, frequency of bowel movements, symptoms associated with bowel movements, Bristol Stool Form rating
   ▪ Ratings of mood subcategories
   ▪ Hunger level
   ▪ Energy level
   ▪ Sleep quantity and quality
   ▪ Skin quality
   ▪ Dietary intake (habitual and acute) (Baseline, Endpoint and Midpoint)
   ▪ Eating behavior - Timing of treatment consumption, Format of consumption, Ranking of main meal energy content (Baseline, Endpoint and Midpoint)
   ▪ Weight (Baseline, Endpoint and Midpoint)
   ▪ Height (Baseline, Endpoint and Midpoint)
   ▪ Waist and hip circumference (Baseline, Endpoint and Midpoint)
   ▪ Medical history (baseline ony)
   ▪ Physical activity level
   ▪ Satisfaction and feasibility of treatment consumption
   ▪ Adherence to treatment and protocol

### Study Design:

The study will take the form of a 6-week randomised controlled trial (6 weeks of active consumption of the allocated treatment). Participants will also complete an onboarding period lasting 3 weeks, where they are contacted by study staff to thoroughly go through the study protocol together, and to provide their baseline measures in the week prior to starting their treatment. Participants will be randomly assigned to one of three dietary intervention arms, which all contain commercially available products or products that are in the process of being brought to market:
1. Prebiotic food topping of the invention - this is the first intervention arm
2. Probiotic capsule containing the single strain Lactobacillus rhamnosus GG (15 billion CFU, Lactobacillus rhamnosus GG, microcrystalline cellulose, hydroxypropyl methylcellulose, sucrose, maltodextrin, magnesium stearate, titanium dioxide (colour), and silicon dioxide). This is commercially available. This treatment will act as the second intervention arm.
3. Salted bread croutons, commercially available from a standard UK supermarket. This will act as a functional control arm. (ingredients: fortified wheat flour (Wheat Flour, Calcium Carbonate, Iron, Niacin, Thiamin), Rapeseed Oil, Sugar, Yeast, Sea Salt, Black Pepper).

Participants will be asked to consume the product of their allocated treatment arm in addition to their habitual diet. They will be asked to ensure their habitual diet remains unchanged throughout the study.

The study will consist of measurement timepoints at baseline (week 0), midpoint (week 4), endpoint (week 6), and weekly (at the end of each treatment phase week, 6 points in total).

During these timepoints all outcomes and data will be measured either through physical human sample collections done at home or through online surveys, completed by the participant.

### Sample Analysis Procedures:

Sample collection and sequencing - At the beginning of the study, participants receive a stool collection kit to collect a stool sample at home. A sample is collected both at baseline and at endpoint. The stool is deposited into a DNA/RNA Shield buffer tube for stability at ambient temperature and sent by standard mail to the analysis laboratory.

DNA Extraction - Total genomic DNA will be isolated using the DNeasy^{®}96 PowerSoil^{®} Pro QIAcube^{®} HT Kit (Qiagen), by following the manufacturer's protocol (homogenization step will be carried out using the Tissue Lyser instrument). Isolated DNA will be quantified using the Quant-iT^{™} dsDNA Assay Kits, broad range (BR) (Invitrogen^{™},Q33130).

Library Preparation - Libraries will be prepared using the Illumina^{®} DNAPrep (Illumina, 20060059) following the manufacturer's protocol and quantified using the Quant-iT^{™} dsDNA Assay Kits, high sensitivity (HS) (Invitrogen^{™},Q33120).

Sequencing - Samples will be processed with Illumina sequencing to a depth of 3.75GB per sample with 150X2 PE reads on the NovaSeq platform and by using the NovaSeq 6000S4 Reagent Kit v1.5. In each sequencing batch the average sequencing depth will beat least 3.75GB and each sample library should receive within 70% of the 3.75GB data target with an Illumina quality score≥Q30.

Metagenome quality control - All metagenomic sequence data undergo a quality control analysis as implemented in the SegataLab preprocessing pipeline. For each sample the pipeline (1) removes low quality reads (quality score<Q20), removes too short reads (<75bp), and reads with more than 2 ambiguous nucleotides; (2) screens and removes reads form the phi X 174 Illumina spike-in genome and human associated reads (hg19); (3) splits and sorts the remaining cleaned reads in forward, reverse and unpaired read files.

### Sample size calculation

Each treatment arm will have 120 participants randomised to it. With three arms, this brings the total number of participants randomised in the study to n=360.

Given previous studies run by this research group, a drop-out rate of 15% has been accounted for, such that a target completion rate of 85% (i.e. n=100per arm) is achieved.

The arm-size of this pilot has been informed by using prior effect estimates from RCTs investigating the effects of prebiotics and probiotics on microbiota composition and human host health (Wastyk et al. Cell, 2021184(16):4137-4153.e14, Liu et al., Clinical Microbiology. 2014;26:1-6, Ukhanova et al. Nutrition. 2014;111(12): 2146-2152).

### Example 3

The ZOE BIOME study (NCT06231706) was a 6-week, parallel design randomized controlled trial in 399 healthy adults in the UK, investigating a simple dietary approach supplementing 30+ whole-food ingredients high in plant polyphenolic compounds, fibre and micronutrients. Participants were randomized to the primary intervention (30g/d) or control (bread croutons; 28g/d; isocaloric functional equivalent) or a daily probiotic (Z. *rhamnosus).* The primary outcome was change in favorable and unfavorable microbiome species compared to control, secondary outcomes included changes in blood metabolites, gut symptoms, stool output, anthropometry, subjective hunger, energy and mood, and sleep. A crossover intervention sub-study was conducted in 34 participants, investigating postprandial glucose responses, subjective hunger, satiety and mood.

A total of 349 male and female participants, mean age 50yrs were included in the analysis (intention-to-treat). Adherence was high overall (over 98%). Following the prebiotic blend, significant improvements were seen in the change and ranking of favorable and unfavorable species, as well as beta diversity (weighted-UniFrac measure), but not in the control or probiotic group. There were significantly greater improvements in self reported outcomes for indigestion, constipation, and energy, following the prebiotic versus probiotic and control. Addition of the prebiotic to a high carbohydrate breakfast resulted in significant improvements in subjective hunger, fullness, and energy (3h iAUC). No other significant differences between groups were observed. This prebiotic blend is a simple strategy that benefits gut microbiome composition, gut symptoms and self-reported energy and hunger over 6 weeks.

In the BIOME (Biotics Influence on Microbiome Ecosystem) study **(****Figure 1****, upper panel),** participants were randomly assigned to consume a prebiotic blend (n = 116; 30g/d), a single-strain probiotic capsule (*L. rhamnosus* 15 billion CFU) (n = 113; 1 capsule/d) or bread croutons (n = 120; 28g/d, isocaloric functional control) for 6 weeks. Study outcomes were assessed using health questionnaires, 24-hr dietary recalls, blood and stool samples collected at baseline and 6-weeks. In the postprandial sub-study **(****Figure 1****, lower panel),** a subset of participants who completed the control arm of the BIOME study were invited to take part in a crossover design postprandial challenge (n=34) in which they consumed a) a test breakfast consisting of white bread, low fat spread and the prebiotic blend (15g) and b) a control breakfast consisting of white bread and low fat spread alone. Meals were consumed in duplicate, in a randomly assigned order with each test day separated by a 2-day washout period. Study outcomes were assessed using continuous glucose monitors (CGMs), visual analogue scales and food records completed on each test day.

### Methods

### Study design

The ZOE BIOME (Biotics Influence on Microbiome Ecosystem) Study was a 6-week parallel-designed randomized controlled trial (RCT) conducted remotely in the UK. In this free-living dietary intervention trial participants were randomly assigned to receive one of three treatments: (i) a prebiotic blend, consisting of whole-food ingredients high in plant polyphenolic compounds, fiber and micronutrients known to exert prebiotic effects on the gut microbiome; (ii) a single-strain probiotic containing *Lacticaseibacillus rhamnosus* GG, provided in capsule form (active control); or (iii) bread croutons, an energy-matched functional equivalent to the prebiotic blend (functional control) **(****Figure 1****, upper panel).** To test the acute health effects of the prebiotic blend, we conducted a postprandial sub-study in a subgroup of participants. In a crossover design, participants consumed test meals in duplicate in a randomly assigned order. Test meals consisted of a) white bread and low fat spread (control) and b) white bread, low fat spread and prebiotic blend **(****Figure 1****, lower panel).**

### Participant selection and randomization

Participants were healthy adults reflective of the average UK population [aged 35-65 y; body mass index (BMI) 18.5 - 40 kg/m²; fibre intake <20g/day). Both sexes (males and females) were eligible for recruitment and sex was determined using self-reported questionnaires with the following question, `Please enter your sex as it was assigned at birth'. Volunteers were excluded from the study if any of the following criteria applied; unable to provide written informed consent through an electronic consent form; unable or unwilling to comply to the study protocol; unwilling to complete study tasks on specified dates; did not complete the Food Frequency Questionnaire (FFQ) at screening; had previously completed the ZOE Nutrition Product; unwilling to consume study treatments ; not based in the UK for the duration of the study; unable to eat the study treatments safely and comfortably (e.g. suffering from inflammatory bowel disease, coeliac disease, Crohn's disease, irritable bowel syndrome, allergies or intolerances, chronic constipation or chronic diarrhoea); BMI of <18.5 kg/m² or >40 kg/m²; following a non-omnivore diet (vegan, vegetarian); high fermented food intake in the previous month (> 7 servings per week); fibre intake > 20g/d in the previous month; treatment with medication or products that may impact study outcome measures in the previous 3 months (e.g. antibiotics, non-topical steroids or other immunosuppressive medicines, biologics, probiotics/prebiotics, metformin, chronic use of non-steroidal anti-inflammatory drugs); use of opiate pain medicine for 8 or more days during the previous 3 months; use of proton pump inhibitors for 8 or more days during the previous 3 months; current smoker; suffered from a heart attack, stroke, or major surgery in previous 2 months; received treatment for cancer in the previous 3 months; were pregnant, breastfeeding or planning pregnancy; were suffering from an eating disorder, type 1 or type 2 diabetes mellitus. Participants were recruited to the trial and screened to assess eligibility against the trial inclusion and exclusion criteria in a two-part process. First, they were invited to complete an online screening questionnaire and FFQ. If eligible according to the initial online screening, participants were enrolled in the study and provided electronic informed consent. Participants were randomly allocated to one of the three treatment groups using a variance minimisation procedure, with sex (male; female), BMI (18.5 - 24.9kg/m²; 25 - 40 kg/m²), and diet quality (Healthy Eating Index; 0-59; 60-100) as stratification variables. The probability of random assignment (pRand) was set to 0.1. Study coordinators informed participants of their allocation to treatment via email. The second part of the screening process was conducted as a video welcome call, during which study coordinators explained trial procedures and confirmed eligibility criteria. Participants that did not meet eligibility criteria were excluded prior to baseline tasks.

### Treatments

Nutrient composition of the prebiotic blend is included in Example 1. The intervention group received the prebiotic blend ("Daily30+"; 30g/d) for 6 weeks. Participants were instructed to consume the treatment by adding it to meals as part of their usual diet. The active control group received a single-strain probiotic containing *Lacticaseibacillus rhamnosus* GG, provided in capsule form and were instructed to consume 1 capsule daily for 6 weeks. The functional control group received bread croutons (Tesco Olive Oil and Sea Salt Croutons; Tesco, UK), an energy-matched functional equivalent to the prebiotic blend. Participants were instructed to consume croutons (28g/d) for 6 weeks by adding them to meals throughout the day.

### Procedures

The study design is summarized in **Figure 1****.** Participants received a study kit via postal delivery containing materials necessary for completing study measurements prior to baseline (week 0) and endpoint (week 7). All procedures were conducted by participants in their homes.

### Baseline week (week 0)

*Health Questionnaires.* Participants completed health questionnaires administered through an online survey (www.typeform.com; www.surveymonkey.com) for collection of baseline and covariate data including subjective ratings of hunger, energy, and mood, gastrointestinal symptoms, anthropometric measurements (waist circumference, body weight), stool frequency and consistency, sleep (quality and quantity), and physical activity.

*Dietary intake.* To capture habitual dietary intake, participants completed an online 24 hour dietary recall (24hr recall; Intake24) on three specified days during the baseline week. Participants were instructed not to report consumption of their assigned treatment via the 24hr recall so we could assess habitual intake only. Adherence to treatment was assessed as described below. The tool prompts participants to list all food and drinks consumed the previous day (from midnight to midnight) using free text entry. Foods were then matched to equivalent items using food composition codes in the Intake24 database, the UK Nutrient Databank. Portion size was reported by participants by selection of a single portion size from a range of options accompanied by food photographs within the online questionnaire. Participants are asked to review their entered items and given the option to enter any further intake before submitting their recall.

*Stool sample collection.* Stool samples for microbiome analysis were collected by participants at home using the Zymo Research Corporation's DNA/RNA ShieldTM Fecal Collection Tube containing a buffer (catalog no. R1101; Zymo Research). The kit contained all the necessary materials for sample collection, along with detailed instructions for use. Participants were instructed to store the sample at room temperature until return by prepaid post to Prebiomics Lab (Trento, Italy).

*Blood sample collection.* Blood samples for metabolomic analysis were collected by participants using the Nightingale Kit^{®}) for remote blood collection (Nightingale Health pic, Finland). Participants were instructed to fast overnight before completing the sample collection in line with kit instructions. Upon completion, sample collection devices were stored in return pouches with desiccant and returned via prepaid postal envelope to a receiving laboratory in the UK. The samples were stored at -80 °C upon receipt until shipping to the Nightingale Health laboratory for analysis (Nightingale Health Pic, Helsinki, Finland).

### Participant monitoring and adherence

Participants confirmed completion of primary baseline study tasks via a survey administered at the end of week 0, and again following completion of endpoint tasks at week 7. Participants who did not report completion of tasks were contacted via telephone or email. Participants in all three arms were asked to self-report adherence to their allocated treatment by completing a questionnaire administered weekly throughout the study period with the following matrix question, `Please fill out the table below to tell us how much of your treatment you consumed each day over the past week' . For the prebiotic blend group, participants were able to select one of the following answer options for each day of the week, `0 scoops, 1 scoop, 2 scoops, >2 scoops for each day of the week' (1 scoop = 15g). For the capsule group, participants were able to select one of the following answer options for each day of the week, `0 capsules, 1 capsule, >1 capsule for each day of the week'. For the control group, participants were first asked if they weighed or counted their croutons before being able to select one of the following answer options for each day of the week, `0 croutons, 1 crouton, 2 croutons, ...22 croutons, > 22 croutons' or `0 grams, 1 gram, 2 grams,..., 28 grams, > 28 grams'. Participants were instructed to maintain their habitual diet during the study; adherence to this instruction was evaluated through 24hr recalls completed at baseline and endpoint.

### Endpoint Measures (week 7)

Endpoint data collection was completed in the 7th week of the study, at which point both groups had consumed their allocated treatments for 6 weeks. All participants completed endpoint measures including health questionnaires, 24HR recall, blood sample and stool sample collection as outlined in the baseline week section above. An additional question was asked at the endpoint only to assess skin improvement.

### Primary outcome measure

The primary outcome of the study was the change in microbiome composition from baseline to the 6-week endpoint, derived from metagenomic analysis of stool samples. Analysis of the primary outcome involved identification of species with a statistically significant difference in terms of relative abundance values from baseline to endpoint, followed by statistical testing of whether the significantly increasing species had significantly higher values of the ZOE Microbiome Ranking 2024 (Cardiometabolic Health)" compared to the values of the significantly decreasing species within each group.

### Secondary outcome measure

Secondary outcomes measures were assessed at baseline and 6-weeks. Dried blood samples were provided for metabolomic analysis of markers lipid profile, fatty acids, glucose control and inflammation. Participants were asked to self-report anthropometric measures including body weight (kg), and waist circumference (cm) which was measured by participants using a measuring tape provided in their study kit. Gut symptoms were assessed using the gastrointestinal symptoms rating scale. Frequency of bowel movements was assessed via a single question "On average, how often do you have a bowel movement?" with the following response options: Once a week or less; Twice a week; Three or four times a week; Five or six times a week; Once a day; Twice a day; Three times a day; Four times a day; Five or more times a day. Stool consistency was assessed via the question "Among the seven choices shown in the image, which stool form is the most common/typical that you experience?" and participants responded by indicating their most common stool consistency on the Bristol Stool Form Scale. Subjective feelings (hunger, energy, happiness, anxiety) were assessed via visual analogue scales. Sleep quality was assessed via the question "During the last 7 days, how would you rate your sleep quality overall?", adapted from a previously validated question, while sleep quantity data was gathered via the question "During the last 7 days, how many hours of actual sleep did you get at night?", with response options (hrs): Less than 5; 5-6; 6-7; 7-8; 8-9; 9-10; 10-11; 11-12; More than 12. Skin quality was determined using the question "If you experience acne, has it improved since starting the BIOME study?" with the following response options: Yes; No; Unsure; Not applicable (endpoint only).

### Blood processing and metabolomic analysis

Samples not meeting quality requirements (device not closed, return pouch not closed or sample not sufficient for analysis) were not included in analysis. A total of 106 metabolites were quantified from blood samples; concentrations for 105 biomarkers were quantified as previously described for venous samples. Briefly, approx. 375 mm2 was taken from the membrane, placed into sodium phosphate buffer (38 mM, pH 7, 10% D20, 0.04 % sodium 3-(trimethylsilyl)propionate-2,2,3,3-d4 (TSP), and 0.02 % sodium azide), and shaken gently for one hour. For each sample, 520 µL of the extract was transferred into a 5 mm NMR tube for the NMR analysis. HbA1c concentration was determined using a Roche cobas c513 analyser with Tina-quant Hemoglobin A1c Third Generation assay. For the analysis, one 6 mm punch was taken from the membrane, placed into a haemolysing reagent (Roche Diagnostics GmbH, Mannheim, Germany), and incubated 30 minutes at room temperature. For each sample, one millilitre of hemolysate was processed in the analyser as per the standard protocol for hemolysate.

### Fecal sampling and microbiome testing

*DNA extraction and sequencing.* DNA was isolated by using the DNeasy 96 PowerSoil Pro QIAcube HT Kit (Qiagen #47021). The DNA was quantified by using the Quant-iTTM 1X dsDNA Assay Kits, BR (Life Technologies, #Q33267) in combination with the Varioskan LUX Microplate Reader (Thermo Fisher Scientific, #VL0000D0). The DNA was diluted in water for the following library preparation.

*Library Preparation and Sequencing.* The sequencing libraries were prepared with the Illumina DNA Prep, (M) Tagmentation (96 Samples, IPB) kit (Illumina, #20060059) in combination with the Illumina^{®} DNA/RNA UD Indexes Set A, B, C, D, Tagmentation (96 Indexes, 96 Samples) (Cat. #20091654, #20091656, #20091658, #20091660) and the amplified libraries were purified with the double-sided bead purification procedure, as described by the Illumina protocol. Then, libraries concentration (ng/pl) were quantified with the Quant-iTTM 1X dsDNA Assay Kits, HS (Life Technologies, #Q33232) in combination with the Varioskan LUX Microplate Reader (Thermo Fisher Scientific, #VL0000D0). In addition, the base pair length (bp) was evaluated by using the D5000 ScreenTape Assay (Agilent, #5067-5588/9) in combination with the TapeStation 4150 (Agilent Technologies, #G2992AA). By knowing both library concentration and base pair length, it is possible to obtain the correct library volume to pool in the same tube in order to achieve optimal cluster density. The library pool was then quantified with the Qubit 1x dsDNA HS kit (Life Technologies, #Q33231) through the Qubit^{®} 3.0 Fluorometer (Life Technologies, #Q33216) and the base pair length (bp) was evaluated as described before. Finally, the library pools were sequenced using the Novaseq X plus platform (Illumina) at an average depth of 3.75 Gb per sample.

### Metagenome quality control and preprocessing

All sequenced metagenomes were preprocessed using the pipeline implemented in https://github.com/SegataLab/preprocessing. Briefly, the pipeline consists of three steps, the first step involves read-level quality control and removes low-quality reads (Q<20), too short reads (length <75bp), and reads with >2 ambiguous nucleotides. The second step screens for contaminant DNAs using Bowtie 25 with the `--sensitive-local' parameter, allowing confident removal of the phi X 174 Illumina spike-in and human-associated reads (hg19 reference human genome release). The last step consists in splitting and sorting the cleaned reads to create standard forward, reverse and unpaired reads output files for each metagenome (average: 35 ± 13 million reads per sample).

### Microbiome taxonomic profiling

Species-level profiling of the samples was performed with MetaPhlAn 4.0. Default parameters were used for MetaPhlAn, with the following database, "mpa_vJan21_CHOCOPhlAnSGB_202103". MetaPhlAn 4 taxonomic profiles were used to assess the presence and contribution of the previously identified 50 positively-associated and 50 negatively-associated species with dietary and cardiometabolic health markers. MetaPhlAn 4 taxonomic profiles were analyzed to compare microbial compositions among participants and to compute an alpha diversity indices, the number of detected species ("observed richness") and the number of detected species taking into account their relative abundance ("Shannon's Diversity Index"). Microbiome taxonomic profiles were also analyzed to compare between microbiome samples dissimilarity (beta-diversity) using the unweighted-UniFrac measure.

### Nutrient intake and diet quality

Daily habitual energy and macronutrient intakes were assessed by averaging the energy and macronutrient intakes from three consecutive 24hr dietary recalls at baseline and 6-weeks. Diet quality was assessed by applying the Healthy Eating Index (HEI).

### Postprandial sub-study

A randomized, controlled, single-blinded 2-phase crossover design study was conducted to determine the effect of the prebiotic blend when consumed alongside a standardized high carbohydrate breakfast (white bread, low fat spread; 60g of available carbohydrate), in comparison to consumption of the high carbohydrate standardized breakfast alone. Study outcome measures were postprandial glucose response, subjective ratings of hunger, satiety, mood and energy and amount consumed at next meal. Participants who completed the control arm of the BIOME study were contacted via email and given the option to take part in the postprandial sub-study. Interested participants were sent a participant information sheet detailing the procedures involved in this additional measurement (Figure 1); and completed a welcome video call with study coordinators for explanation of study procedures. Participants provided electronic informed consent prior to enrolment in the sub-study. Blinding of participants to the intervention was not possible due to the nature of the test meals (whole foods, for which a placebo that is void of nutrients/properties of interest, but physically similar, is not possible to create). Study coordinators and analysts were blinded to treatment allocation by coding test meals in all data collection documents and databases as "Test meal A" (control) and "Test meal B" (intervention). Treatment sequence was randomized using online software (https://www.sealedenvelope.com/) by an independent researcher. This was performed by randomly assigning participants to one of six possible meal sequences (AABB, ABAB, ABBA, BBAA, BABA, BAAB) using the block randomization service (block sizes of 12), stratified by biological sex (male, female). Participants were informed of meal sequence via a printed instruction leaflet that was included in their study kit. Study kits were sent to participants via post ahead of the study start date, and contained the following items; continuous glucose monitor (CGM; Abbott Diabetes Care, Alameda CA, USA), food weighing scales (Arc Digital Kitchen Scale; Salter, UK), the prebiotic blend, plastic scoop (capacity 15g), study guide, questionnaire booklet, prepaid return envelope, meal sequence leaflet. To ensure fresh food items were within sell-by dates participants were given instructions of exact fresh food items to purchase from local supermarkets; white bread (Warburtons Farmhouse White Bread, Warburtons UK) and low fat spread (Flora Lighter Spread, Flora UK); and received reimbursement for these products. The study took part of a 10-day intervention period. Test meals were consumed in duplicate over 4 test days (days 1, 4, 7 and 10), with each test day separated by a 2-day washout period.

Participants were instructed to apply their CGM on the upper non-dominant arm, the day before their first test day (day 0). An adhesive patch was applied on top of the monitor to ensure secure attachment (Sourceful, Manchester, UK). The CGM was worn for the duration of the study period (10 days). Participants were given instructions to follow in the 24 hours ahead of each test day; avoid drinking alcohol, strenuous exercise and fast for 8 hr (no food or drink except water). On the morning of each test day participants were instructed to avoid smoking and use of tobacco products and consume a standardized amount of water. Baseline measures were conducted via a questionnaire booklet immediately before consumption of the test meal, and included subjective ratings of hunger, satiety, energy, mood and alertness. Participants were then instructed to consume test meals within a 15 min time window (0-15 min). Following consumption of the test meal, participants were asked to fast for 3 hr, avoid smoking or use of tobacco products, avoid strenuous exercise, avoid taking medications and were permitted to consume a standardized amount of water during this time. Further questionnaires were completed at 15, 60, 120 and 180 min. After completion of the 3 hr post-meal fast, participants reported the time they consumed their next meal and details of food consumed in their questionnaire booklet. When all four test days had been completed, participants returned their questionnaire booklets via prepaid return envelope.

### Test meals

A standardized high carbohydrate breakfast was designed, consisting of white bread (128g; 57.6g carbohydrate (CHO), 3.2g fat, 11.5g protein, 2.9g fibre) and low fat spread (10-15g; 0.1g CHO, 3.5g fat, 0.1g protein, fibre not reported). The control test meal consisted of the standardized breakfast meal alone. The intervention test meal consisted of the standardized breakfast meal, in combination with the prebiotic blend (30g; 5.3g CHO, 7.7g fat, 5.5g protein, 9.0g fibre; see Example 1).

### Continuous glucose monitoring

Interstitial glucose was measured every minute and aggregated into 15 minute readings, using Freestyle Fibre Pro CGM (Abbott Diabetes Care, Alameda, CA, US). Glucose measurements were downloaded from the CGM onto the FreeStyle FibreFink mobile application (Abbott) by scanning the device with a smartphone containing the application download. Participants were provided with login details that linked their FibreFink application to the study practice account for retrieval of outcome data. Participants applied CGM devices 24hr before their first test meal, and data for the first 12 hr of CGM usage were discarded prior to analysis.

### Primary outcome

The primary outcome of the postprandial sub-study was the difference in peak postprandial glucose concentration (C-Max) between the intervention and control test meals assessed using CGM-derived glucose concentration data.

### Secondary outcomes

Additional CGM derived metrics indicative of postprandial glycaemic response were analyzed as secondary outcomes, including the difference in 2-h incremental area under the curve (2-h iAUC), time to max concentration (T-max), 2-3h dips below baseline (dips), and Time Course Analysis (i.e. Meal*Time interactions). Subjective ratings of satiety (hunger, fullness, desire to eat, satisfaction, prospective consumption), energy, mood (happiness, anxiety) and alertness were assessed using visual analogue scales (0-100mm). Time to next meal (min) and energy and macronutrient intake at next meal were assessed by a food diary included in the participant questionnaire booklet.

### Sample size calculations

The primary outcome of the BIOME study was based on the change in relative abundances of microbiome species previously identified for their associations with markers of cardiometabolic health, from baseline to the 6-week endpoint. The study was powered to detect differences between groups in the primary outcome measure, using proprietary data collected within the ZOE commercial product. Based on a two-sided significance level (α) of 0.05, with 85% power, a sample size of 102 participants per group (306 participants in total) was calculated. An anticipated attrition rate of 20-25% was applied based on rates in previous studies conducted by the research group, resulting in a total of 133 participants per group (399 participants in total).

To ensure sufficient power to detect an effect of the dietary intervention on postprandial glucose response, a second sample size calculation was performed. The postprandial sub-study was powered to detect changes in the primary outcome (C-max) using pilot data collected during the ZOE PREDICT 1 study (unpublished data). A within patient standard deviation of the difference in peak glucose concentration following a high carbohydrate versus a high fibre breakfast test meal was calculated (1.19). Based on a two-sided significance level (α) of 0.05, with 80% power, a minimal detectable difference of 0.582, a sample size of 35 participants was calculated. Based on previous similar studies conducted by our research group, we anticipate a dropout rate of 15%, resulting in a total of 40 participants being required to take part in the crossover sub-study.

### Results

### Study participant characteristics:

A total of 8,017 volunteers were screened for eligibility. 399 participants were randomly assigned to the primary intervention (prebiotic blend; n = 133), active control (probiotic capsule; n = 133) or functional control (bread croutons; n = 133) groups. 50 participants did not met selection criteria following randomisation, resulting in 349 participants included in the intention-to-treat analysis set. Recruitment, randomisation and reasons for exclusion are summarized in the Consolidated Standards of Reporting Trials (CONSORT) diagram **(****Figure 2****).** In the acute postprandial sub-study, 40 participants who completed the control arm of the chronic phase, and opted to take nart were randomly assigned to one of six meal orders. A total of 34 participants completed the postprandial intervention phase and were included in the analysis.

At baseline, there were no significant differences of participant characteristics per randomized group **(Table 3).** In total 75.4 % of participants were female and had a median age of 51.4 years (inter-quartile range, IQR 12.35) and body mass index (BMI) of 25.9 kg/m² (IQR 5.9). The ethnicity of individuals was primarily white (93.4%), and the majority achieved a university or postgraduate degree (69.6%). Overall diet quality was high (Healthy eating index (HEI) 69.0, s.d . 8.S), and participants achieved 135 minutes (IQR 180) of moderate-vigorous physical activity per week. Within the total sample, participants had median fibre intake of 16.4 g/d (IQR 5.3), and consumed 0.3 servings (IQR 0.4) of fermented food per day. There were no smokers among participants. Variables known to impact study outcome measures related to microbiome and metabolic health were compared between groups at baseline and no significant differences were found (age, sex physical activity or HEI) **(Table 3).**

**Table 3 - BIOME study participant disposition**

| | **Total cohort (n = 349)** | | **Prebiotic blend (n = 116)** | | **Probiotic (n = 113)** | | **Control (n = 120)** | |
|---|---|---|---|---|---|---|---|---|
| **Sex n, %** | | | | | | | | |
| Female | 263 | 75.4 | 87 | 75 | 86 | 76 | 90 | 75 |
| Male | 86 | 246 | 29 | 25 | 27 | 24 | 30 | 25 |
| **Age (years)^{a}** | 51.4 | 12.4 | 51.5 | 11.4 | 51.6 | 11.4 | 50.1 | 13.4 |
| **Ethnicity n, %** | | | | | | | | |
| White | 326 | 93.4 | 110 | 95 | 104 | 92 | 112 | 93 |
| Asian, Asian British or Asian Welsh | 11 | 3.15 | 2 | 2 | 3 | 3 | 6 | 5 |
| Black, Black British, Black Welsh, Caribbean or African | 6 | 1.72 | 2 | 2 | 4 | 4 | 0 | 0 |
| Mixed or Multiple ethnic groups | 3 | 0.86 | 1 | 1 | 1 | 1 | 1 | 1 |
| Unknown | 3 | 0.86 | 1 | 1 | 1 | 1 | 1 | 1 |
| **Education status n, %** | | | | | | | | |
| Up to and including GCSE (or equivalent) | 24 | 6.9 | 10 | 8.6 | 5 | 4.4 | 9 | 7.5 |
| A Level (or equivalent) | 40 | 11.5 | 15 | 12.9 | 12 | 10.6 | 13 | 108 |
| Higher Vocational training (e.g. Diploma, NVQ4) | 31 | 8.9 | 6 | 5.2 | 16 | 14.2 | 9 | 7.5 |
| University or postgraduate degree | 243 | 696 | 80 | 69.0 | 76 | 67.3 | 87 | 72.5 |
| Other | 11 | 3.2 | 5 | 4.3 | 4 | 3.5 | 2 | 1.7 |
| **BMI (kg/m2)^{a}** | 25.9 | 5.9 | 26.0 | 6.7 | 25.7 | 5.6 | 25.9 | 5.5 |
| **HEI Score (0-100)^{b}** | 69 | 9 | 69 | 7 | 71 | 9 | 68 | 9 |
| **Physical activity (mins/week)^{a}** | 135 | 180 | 142.4 | 180 | 140 | 180 | 120 | 187.5 |
| **Smoking status (non-smoking) n, %** | 349 | 100 | 116 | 100 | 113 | 100 | 120 | 100 |

| | | | | | | | | |
|---|---|---|---|---|---|---|---|---|
| ^{a}Data are median, IQR unless otherwise stated. ^{b}Data are mean, s.d.; No significant difference between the prebiotic blend vs control, or prebiotic blend vs probiotic. Physical activity is minutes of moderate-vigorous intensity activity per week. | | | | | | | | |

### Adherence to treatment and habitual diet:

Self-reported adherence to assigned treatments, in those completing all weekly check-in questionnaires, in the intention to treat (ITT) cohort (n = 323) was high overall (99.0%) and across groups; prebiotic blend (98.3%), probiotic (99.6%), control (99.1%). Participants were instructed to maintain a habitual background diet, which was monitored by completion of 24 hr dietary recalls at baseline and 6-weeks. Participants in the prebiotic blend group had marginally greater energy intake from total sugar (mean ± s.d.; 16% ± 7%) in comparison to the probiotic group (15% ± 5%; *p* = 0.04, ANCOVA). There were no other differences in energy or macronutrient intake between groups.

### Microbiome analysis:

As basic traditional gut microbiome information, we calculated the weighted-UniFrac measure of beta-diversity and both species richness and Shannon alpha diversity measures. For the weighted-UniFrac measure, there was no visual separation between the baseline microbiome composition across groups (PERMANOVA p-value = 0.5841), while endpoint microbiome compositions show significant differences, in particular for the prebiotic blend group (PERMANOVA p-value = 0.0198). PERMANOVA analysis performed within each group comparing baseline with endpoint microbiome composition, showed significant differences only for the prebiotic blend (prebiotic blend p-value = 0.0297, probiotic p-value = 0.3267, control p-value = 0.0594). Shannon's diversity index tended to increase across all three groups (but significant only in probiotic group, Wilcoxon's p-value = 0.0203), while richness showed a slight change of detected species at endpoint across all three groups (decrease significant only in the prebiotic blend group, Wilcoxon's p-value = 0.0008).

The pre-specified primary outcome of the study was the improvement of the microbiome composition measured using the ranking of prevalent microbiome species associated with cardiometabolic health. This ranking was developed by our group, to identify and prioritize microbial species most likely affecting host health either in a positive or negative way in over 34,000 individuals. To assess the impact of the three different supplements, in each group, we identified the prevalent species (classified using species level genome bins) at both baseline and at 6-week and then tested if the relative abundance values between the two time points were significantly different (paired Wilcoxon signed-rank test; FDR adjusted p-values < 0.01). We identified n = 57 species that were significantly different in the prebiotic blend group); n = 4 species in the probiotic group; and n = 14 species in the control group in the ITT cohort. Of these significant species, we then distinguished them according to whether they increased or decreased their relative abundance at 6-weeks, and tested whether the significantly increasing (or decreasing) species had significantly different "ZOE Microbiome Ranking 2024 (Cardiometabolic Health)". Rank values closer to 0 are indicative of favorable species associated with better health prediction, while ranks closer to 1 indicate unfavorable species associated with poorer health outcomes. In the prebiotic blend group, the median rank of decreasing species (0.659) was significantly higher (unfavorable) than the median rank of increasing species (0.408, favorable), indicating that the prebiotic supplementation impacts the microbiome by increasing species associated with favorable cardiometabolic health markers while decreasing those associated with less favorable cardiometabolic health markers (p = 0.007; Mann-Whitney U-test; **Figure 3A****, 3C).** This significant effect was not seen in either the probiotic group (median rank (decreasing) = 0.604; median rank (increasing) = 0.694; p = 0.50) or the control group (median rank (decreasing) = 0.604; median rank (increasing) = 0.519; p = 0.55) **(****Figure 3A****).** In addition to their significant association with the ZOE Microbiome HEALTH ranking, it was found that the significantly increasing and decreasing species showed different prevalence patterns between baseline and 6-weeks. Increasing species tended to resemble or slightly increase their prevalence (from 74.1% to 76.9% median prevalence, Wilcoxon paired test p = 0.00053), while decreasing species tended to be much less prevalent (from 61.3% to 44.3% median prevalence, Wilcoxon paired test p = 0.000057) **(****Figure 3D****).**

To explore the effect of the prebiotic blend on microbiome species associated with markers of diet quality, we evaluated the identified significant species according to their "ZOE Microbiome Ranking 2024 (Diet)". In the prebiotic blend group, the median rank of decreasing species (0.686, unfavorable) was significantly higher than the median rank of increasing species (0.323, favorable), indicating that increasing species were those associated with favorable diet quality indices while decreasing species were those associated with less favorable diet quality indices (p < 0.001; Mann-Whitney U-test; **Figure 3B****).** This significant effect was not seen in either the probiotic group (median rank (decreasing) = 0.641; median rank (increasing) = 0.410; p = 1.0) or the control group (median rank (decreasing) = 0.546; median rank (increasing) = 0.410;p = 0.55) **(****Figure 3B****).**

Finally as a measure of adherence we investigated the presence of the probiotic *L*. *rhamnosus* across the groups at baseline and 6-weeks. Only the probiotic group showed a significantly larger number of individuals from which we were able to identify *L*. *rhamnosus* in their gut microbiome at 6-weeks (from 5 to 58) **(Figure** 3F).

### Secondary outcomes:

*Self-report*: Subjective ratings of energy and mood were measured using visual analogue scales (0-100mm) and gastrointestinal symptoms were assessed using the Gastrointestinal Symptom Rating Scale (GSRS) at baseline and 6-weeks. For the primary comparison (prebiotic vs control) a greater proportion of participants reported improvements in energy (50.5% vs 37.3%); happiness (44.6% vs 30%); severity of indigestion domain symptoms (55.1%, IQR vs 36.4%); severity of constipation domain symptoms (34.6%, IQR vs 24.5%), severity of flatulence (37.4% vs 23.6%); severity of heartburn (17.8% vs 11.8%); and total gastrointestinal symptoms (69.2% vs 56.4%) following the prebiotic in comparison to control. Estimates of effect size are reported in **Figure 4****.** Stool frequency was significantly different following prebiotic blend in comparison to control at endpoint, with a smaller proportion of the blend group reported stool frequencies of "Three or four times per week" (4%), than following the functional control (14.2%) **(****Figure 6****).** Anthropometric (self reported) measures (waist circumference and weight), stool consistency, acne, sleep quality and quantity, and remaining subjective emotions and gastrointestinal symptoms did not differ significantly between groups **(****Figure 6****).** For the secondary comparison (prebiotic vs probiotic), there were greater reductions in severity of constipation (individual symptom, -0.26 vs 0.04; p = 0.007), and hunger (-0.86 vs -0.44; p = 0.006) **(****Figure 6****)** following the prebiotic in comparison to probiotic. There were no other clinically significant differences between groups **(****Figure 6****).**

*Metabolomics*: For both the primary comparison of prebiotic blend vs. control and the secondary comparison of the prebiotic blend vs probiotic, there were no clinically significant differences between groups for any blood metabolites at beginning and end.

### Post-hoc analysis:

Pre-planned subgroup analysis was performed on core metabolites indicative of lipid profile (apolipoproteinB, ApoB; LDL-cholesterol, LDL-C; Triglycerides, TG), and inflammation (glycoprotein acetyls, GlycA). In those with highest metabolite concentrations at baseline (top tertile), there were no differences between groups observed for changes in these outcome measures. Within group analysis showed small but statistically significant reductions in the prebiotic group for ApoB (-0.06 mmol/L, p = 0.003; n = 33), LDL-C (-0.22 mmol/L; p = 0.0005; n = 32) and GlycA (-0.04 mmol/;p = 0.03; n = 29). In the probiotic group there were significant reductions in GlycA (-0.04 mmol/; p = 0.03; n = 34), LDL-C (-0.04 mmol/; p = 0.15; n = 30), and TG (-0.2 mmol/; p = 0.02; n = 30). There were no significant changes in the control group from baseline to 6-weeks.

In participants who reported baseline gastrointestinal symptoms (severity score >2), there were significant differences between the prebiotic blend and control for improvements in severity of rumbling stomach (mean (95%CI); -1.0 (-1.2, -0.8) vs -0.5 (-0.8, -0.3); p = 0.008) and constipation -1.0 (-1.2, -0.8) vs -0.45 (-0.8, -0.1); p = 0.03).

In addition there was a significantly greater proportion of participants reporting improved sleep quality following the prebiotic blend in both the primary comparison (34.9% vs control 20%; p = 0.01) and the secondary comparison (vs probiotic 19.6%; p = 0.01). No other significant differences were observed.

### The impact of the dietary intervention on postprandial glucose responses, energy, hunger and satiety:

In the postprandial cross-over sub-study comparing a high carbohydrate standardized breakfast with or without the prebiotic blend, there were no significant differences in postprandial glycaemia. However, the addition of the prebiotic blend to the test meal resulted in significantly greater subjective fullness (41.5%), meal satisfaction (21.6%), and energy (43.3%) and lower hunger (-16.9%), desire to eat (-70.9%) and prospective consumption (-54.2%), p<0.05 for all; **Figure 5****.** Energy and macronutrient intake at the next meal was not significantly different following test meals; with the exception of fibre for which there was a small but significantly lower intake following the high carbohydrate meal with the prebiotic blend, in comparison to the high carbohydrate meal alone (mean±s.d.; 4.1g±3.1 vs 5.1g±3.7; p = 0.045). Time to the next meal did not differ significantly following test meals. There were no differences in time course analysis between meals for glucose metrics or subjective outcomes.

### Discussion

Alternative interventions are needed to improve dietary intake, for the prevention of diet-related disease and maintenance of health. This randomized controlled trial investigated the effect of a prebiotic blend, designed to be added to the diet to improve intakes of fibre and diverse plants, on gut microbiome composition and associated health outcomes in healthy adults. It was found that daily consumption of the prebiotic blend improved gut microbiome composition, specifically species previously associated with markers of cardiometabolic health and diet quality. The prebiotic blend also resulted in improvements in gastrointestinal symptoms and subjective feelings of energy and mood demonstrating another potential benefit of the intervention on overall health and well-being. A postprandial crossover sub-study was carried out to test the acute health effects of the prebiotic blend, powered to detect changes in glycaemic response. While there was no effect of the intervention on the primary outcome in the postprandial study, the results for subjective ratings of hunger, satiety and energy indicate that the prebiotic blend may also benefit acute health potentially due to its nutrient content, specifically fibre (9.0g per 30g serving) and protein (5.5g per 30g serving).

The gut microbiome is increasingly recognized as an important mediator of the impact of diet on human health. Plant-based diets and increased intakes of plant food groups such as wholegrains, have been shown to modulate gut microbiome composition resulting in positive health outcomes in both healthy adults and those at increased risk of adverse cardiometabolic health. However, despite decades of public health messaging, recommendations to increase consumption of fruits, vegetables and wholegrains remain unmet, potentially due to increased burden of preparation of fresh foods, poor nutritional education and demanding modern lifestyles. Diversity of plants in the diet is another factor that is increasingly proposed for its impact on gut microbiome composition and health, due to increased presence of diverse fibre in addition to adequate provision of other macro- and micronutrients and plant-based bioactives such as polyphenols. In a large study of over 1500 individuals from across three countries, the diversity of plants consumed was found to be associated with microbiome diversity, to a greater extent than having a vegan/vegetarian dietary pattern (McDonald et al., American Gut: an Open Platform for Citizen Science Microbiome Research, mSystems 3, 10.1128/msystems.00031-18 (2018)), suggesting that increasing diversity can have beneficial effects on the microbiome across individuals, regardless of dietary pattern. Simple plant-diverse dietary interventions that target the gut microbiome therefore have the potential to improve diet-related health outcomes. To test this hypothesis, we designed a blend of 30 whole plants including fruits/vegetables (6), mushrooms (8), herbs (3), nuts (3), seeds (6), spices (2), wholegrains (2), chosen for their content of diverse fibre, micronutrients and polyphenols and prebiotic compounds.

In this trial we have demonstrated the primary hypothesis that this blend would exert a prebiotic effect on gut microbiome composition. Its consumption for 6-weeks led to significant shifts in species previously identified as either favourable or unfavourable based on their association with cardiometabolic health outcomes, and diet quality. Specifically, the prebiotic blend resulted in an increase in both relative abundance and prevalence of favourable bacterial species, and a decrease in unfavourable species - an effect that was not seen in either the functional control or probiotic groups.

The blend is rich in nutrients shown to impact cardiometabolic health outcomes, including diverse fibres, polyunsaturated fatty acids and polyphenols. Dietary interventions that impact gut microbiome composition may have benefits to immune health due to the close relationship between these biological systems. Therefore, it was hypothesized that consumption of the blend for 6-weeks may benefit circulating metabolites related to lipid profile (apolipoprotein B, LDL-cholesterol, triglycerides), and inflammation (GlycA). Following the prebiotic blend, we saw improvements in gut microbiome species associated with cardiometabolic health, and improvements in GlycA and ApoB concentrations in a sub-group with highest metabolite concentrations before the intervention. Our findings suggest that regular consumption of the blend by individuals at greater cardiometabolic risk may have benefits for metabolic and immune health. Similar results were seen following consumption of the probiotic, indicating the prebiotic blend can exert benefits similar to those seen using interventions targeting the gut microbiome.

The results of the postprandial study support the findings from the 6-week intervention, and indicate that improvements in subjective outcome measures such as energy and hunger occur with both acute and chronic consumption. The current food landscape in the UK and countries where a western dietary pattern is prevalent, is one of excessive consumption of hyper-palatable foods high in sugar, salt and saturated fats, and low in fibre that typically do not have a high satiating capacity therefore encouraging overconsumption. The results of this postprandial study demonstrate that a simple plant-diverse dietary addition, rich in fibre and plant-based protein has the potential to decrease hunger and increase satiety.

Self-reported adherence to the intervention in the prebiotic blend group was extremely high, indicating this intervention was feasible and well tolerated by participants. While shifts in overall dietary pattern may stand to induce broader health effects, convenient dietary strategies that are low-burden have potential to impact diet and therefore diet-related health outcomes in the shorter term and in individuals with demanding lifestyles for whom larger dietary shifts may be unattainable.

In conclusion, the simple, convenient fibre-rich plant diverse blend of whole food ingredients can be added to the diet or to replace less nutrient dense alternatives that are designed to add flavour, but are typically nutrient poor. The prebiotic blend therefore encourages improvement in diet quality and provides promising additional benefits to microbiome composition, subjective energy and hunger, and possibly health.

### ASPECTS OF THE INVENTION

1. A food composition comprising:
   (a) at least two sources of dietary fibre, wherein the sources of dietary fibre are obtained from two or more of: flaxseed, chia seed, sunflower seeds, red lentil flakes, chicory root inulin fibre, nutritional yeast flakes, almonds, hazelnuts, walnuts, brazil nuts, puffed quinoa, puffed buckwheat and red beetroot flakes;
   and at least 2 of the following:
   (b) at least one source of polyphenols, wherein the source of polyphenols is obtained from one or more of mushroom powder, thyme, onion powder, parsley, rosemary, turmeric, cumin, flaxseed, baobab powder, grape seed powder, garlic, buckthorn powder, red beetroot flakes, and carrot flakes;
   (c) at least one plant protein;
   (d) at least one source of alpha linolenic acid (ALA); and
   (e) at least one source of phytosterols, wherein the source of phytosterols is obtained from one or more of sunflower seeds, pumpkin seeds, hemp seed, almonds, hazelnuts, walnuts, and brazil nuts,
   wherein the composition comprises at least one nut or seed.
2. The composition of any aspect 1, which does not contain a sweetener.
3. The composition of any one of the preceding aspects, which is savoury.
4. The composition of any one of the preceding aspects, which is suitable for sprinkling on top of a food item.
5. The composition of any one of the preceding aspects, wherein the structural matrix of at least one of (a)-(e) is maintained.
6. The composition of any one of the preceding aspects, wherein at least one of (a)-(e) is nibbed so as to maintain its structural matrix.
7. The composition of any one of the preceding aspects, which contains ingredients that are not powdered, such as ingredients that have a particle size of at least 1mm, 2mm or 5mm.
8. The composition of any one of the preceding aspects, which is a wholefood composition.
9. The composition of any of the preceding aspects, which does not contain an anti-caking agent.
10. The composition of any of the preceding aspects, which does not contain an additive.
11. The composition of any one of the preceding aspects, which is a prebiotic food supplement.
12. The composition of any one of the preceding aspects, which comprises at least 3, 4 or 5 different sources of each of (a)-(e).
13. The composition of any one of the preceding aspects, wherein the mushroom powder comprises one or more of Lions Mane, Reishi, Chaga, Shiitake, Cordyceps, Maitake, Tremella.
14. The composition of any one of the preceding aspects, wherein the plant protein is obtained from one or more of the group consisting of: lentils, quinoa, buckwheat, chia seeds, hemp seeds, almonds, chickpeas and nutritional yeast flakes, optionally wherein the plant protein is obtained from one or more of: puffed quinoa and puffed buckwheat.
15. The composition of any one of the preceding aspects, wherein the source of ALA is obtained from one or more seeds, optionally wherein the source of ALA is selected from the group consisting of: flaxseed and chia seeds.
16. A food composition comprising or consisting of at least 15 of the following: flaxseed, chia seeds, sunflower seeds, pumpkin seeds, hemp seeds, red lentil flakes, grape seed, almonds, hazelnuts, walnuts, chicory root inulin fibre, puffed quinoa, white mushroom, thyme, onion, parsley, turmeric, cumin, Lion's Mane, Reishi, Chaga, Shiitake, Cordyceps, Maitake, Tremella, rosemary, garlic, red beetroot flakes, carrot flakes, nutritional yeast flakes, baobab powder, and buckthorn.
17. A method of supplementing the dietary needs of a human, comprising administering to the human the food composition of any one of aspects 1-16.
18. A method of benefitting the gut microbiome of a human using the food composition of any one of aspects 1-16, wherein the food composition is ingested by the human.
19. A method of reducing the risk of disease in a human or improving the general well-being of a human using the food composition of any one of aspects 1-16, wherein the food composition is ingested by the human.
20. A method of controlling or treating obesity in a human using the food composition of any one of aspects 1-16, wherein the food composition is ingested by the human.
21. A method of reducing inflammation in a human using the food composition of any one of aspects 1-16, wherein the food composition is ingested by the human, optionally wherein reducing inflammation comprises reducing inflammatory markers.
22. A method of treating gastrointestinal symptoms of a human, for example, constipation and bloating, using the food composition of any one of aspects 1-16, wherein the food composition is ingested by the human.
23. A method of improving one or more of the following: bowel function of a human, mood of a human, skin quality of a human, satiety of a human, glycaemic profile of a human, lipaemic profile of a human, or sleep quality of a human, using the food composition of any one of aspects 1-16, wherein the food composition is ingested by the human.
24. A method of strengthening the immune system of a human using the food composition of any one of aspects 1-16, wherein the food composition is ingested by the human.
25. The method of any one of aspects 17-24, wherein a daily dosage of about 15g of the food composition is ingested by the human, optionally wherein the daily dosage comprises about 5g of fibre, and/or wherein the composition is sprinkled on top of a food item.

## Claims

1. A food composition comprising or consisting of at least 15 of the following ingredients: flaxseed, chia seeds, sunflower seeds, pumpkin seeds, hemp seeds, red lentil flakes, grape seed, almonds, hazelnuts, walnuts, chicory root inulin fibre, puffed quinoa, white mushroom, thyme, onion, parsley, turmeric, cumin, Lion's Mane, Reishi, Chaga, Shiitake, Cordyceps, Maitake, Tremella, rosemary, garlic, red beetroot flakes, carrot flakes, nutritional yeast flakes, baobab powder, and buckthorn.

2. The composition of claim 1, wherein the structural matrix of at least one ingredient is maintained.

3. The composition of claim 1 or 2, wherein:
(a) the composition is suitable for sprinkling on top of a food item; and/or
(b) the composition contains ingredients that have a particle size of at least 1mm, 2mm or 5mm.

4. The composition of any one of the preceding claims, wherein:
(a) the composition is savoury;
(b) the composition does not contain a sweetener;
(c) the composition does not contain an anti-caking agent; and/or
(d) the composition does not contain an additive.

5. The composition of any one of the preceding claims, which is a wholefood composition and/or a prebiotic food supplement.

6. A non-therapeutic method of supplementing the dietary needs of a human, comprising administering to the human the food composition of any one of claims 1-5.

7. A non-therapeutic method of benefitting the gut microbiome of a human using the food composition of any one of claims 1-5, wherein the food composition is ingested by the human.

8. A non-therapeutic method of reducing the risk of disease in a human or improving the general well-being of a human using the food composition of any one of claims 1-5, wherein the food composition is ingested by the human.

9. A non-therapeutic method of improving one or more of the following: bowel function of a human, mood of a human, skin quality of a human, satiety of a human, glycaemic profile of a human, lipaemic profile of a human, sleep quality of a human, or immune system of a human using the food composition of any one of claims 1-5, wherein the food composition is ingested by the human.

10. The food composition according to any one of claims 1-5, for use in a method of controlling or treating obesity in a human, wherein the food composition is ingested by the human.

11. The food composition according to any one of claims 1-5, for use in a method of treating gastrointestinal symptoms of a human, wherein the food composition is ingested by the human.

12. The food composition for use according to claim 11, wherein the gastrointestinal symptoms are selected from: constipation and bloating.

13. The food composition according to any one of claims 1-5, for use in a method of strengthening the immune system of a human, wherein the food composition is ingested by the human.

14. The non-therapeutic method of any one of claims 6-9, or the food composition for use according to any one of claims 10-13, wherein a daily dosage of about 15g of the food composition is ingested by the human.

15. The non-therapeutic method or food composition for use according to claim 14, wherein the daily dosage comprises about 5g of fibre, and/or wherein the composition is sprinkled on top of a food item.
